(19) Europäisches Patentamt
European Patent Office
Office européen des brevets

(11) EP 2 392 555 A2

(12) **EUROPEAN PATENT APPLICATION**
published in accordance with Art. 153(4) EPC

(43) Date of publication:
**07.12.2011 Bulletin 2011/49**

(21) Application number: **10736046.3**

(22) Date of filing: **29.01.2010**

(51) Int Cl.:
***C07C 15/56*** *(2006.01)* ***H01L 29/786*** *(2006.01)*
***H01L 31/042*** *(2006.01)*

(86) International application number:
**PCT/KR2010/000572**

(87) International publication number:
**WO 2010/087655 (05.08.2010 Gazette 2010/31)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.01.2009 KR 20090006781**
**29.01.2009 KR 20090006783**
**29.05.2009 KR 20090047438**

(71) Applicant: **Korea Research Institute of Chemical Technology (KRICT)**
**Yuseong-Gu**
**Daejeon 305-343 (KR)**

(72) Inventors:
- **YOON, Sung Cheol**
**Yongin-si**
**Gyeonggi-do 448-130 (KR)**
- **NAM, So Youn**
**Daejeon 302-775 (KR)**
- **LIM, Hyun Seok**
**Daejeon 300-120 (KR)**
- **LEE, Jaemin**
**Daejeon 305-509 (KR)**
- **LIM, Jongsun**
**Daejeon 305-804 (KR)**
- **KIM, Dong Wook**
**Daejeon 305-751 (KR)**
- **KANG, Yongku**
**Daejeon 305-707 (KR)**
- **LEE, Chang Jin**
**Daejeon 305-762 (KR)**
- **JUNG, Jae Wook**
**Daejeon 302-280 (KR)**

(74) Representative: **Stolmár Scheele & Partner**
**Blumenstraße 17**
**80331 München (DE)**

# (54) FULLERENE DERIVATIVES AND ORGANIC ELECTRONIC DEVICE COMPRISING THE SAME

(57) The present invention relates to fullerene derivatives and an organic electronic device using the same, and more specifically, to a novel fullerene derivative incorporating an aromatic fused ring compound and to an organic electronic device with excellent electrical properties by employing the fullerene derivative. In more detail, the novel fullerene derivative incorporating an aromatic fused ring compound according to the present invention exhibits excellent solubility in organic solvents and has a high electrochemical electron mobility and a high LUMO energy level, thereby making the fullerene derivative a suitable material for organic solar cells featuring a high open circuit voltage (Voc) and an improved energy conversion efficiency, or applicable for use in organic electronic devices such as organic thin film transistors.

【FIG. 1】

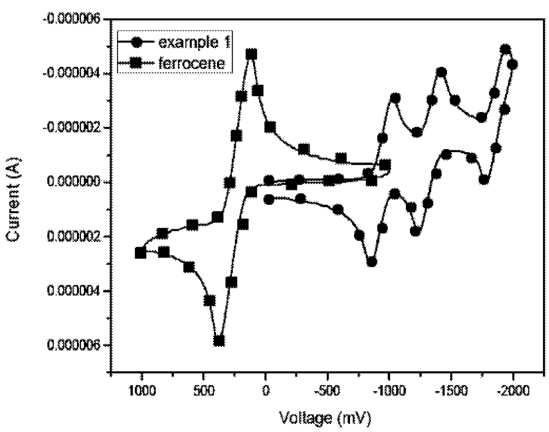




Printed by Jouve, 75001 PARIS (FR)

## Description

[Technical Field]

The preset invention relates to a novel fullerene derivative as an organic semiconductor material, and an organic electronic device comprising the same, and more particularly to an organic semiconductor material of a fullerene derivative, to which an aromatic fused ring is linked, and an organic electronic device comprising the same.

[Background Art]

In the past 10 years, development of organic materials exhibiting semiconductor properties, and also various kinds of applied studies using the same has made progress. An area of applied study using an organic semiconductor, such as electromagnetic wave shielding layers, capacitors, OLED displays, organic thin film transistors (OTFTs), solar cells, memory devices using multi-photon absorption, is expanding continuously. Among them, a field of OLED functions as a catalyst of activating applied study using an organic matter since commercialization of large-sized displays is just around corner. In addition, starting circuits for active driving of OLED, and also, organic semiconductor thin film transistors, which are expected to be used even in application of next-generation smart cards, are fast growing. After electric generation characteristics using an organic semiconductor as an active layer are presented, application thereof as a laser diode also has been receiving much attention, again. The organic materials are remarkably cheaper than non-organic materials in manufacturing cost of a device, and thus a revolution is foretelling in a future solar cell market.

A study on the organic semiconductor thin film transistor has been researched since 1980, but recently, the study is progressing in earnest over the world. An electronic circuit board, which can be manufactured by a simple process and a low cost, unbreakable at impact, and flexible or foldable, is expected to be an essential element for future industries. Therefore, development on an organic transistor satisfying these needs is emerging as a field of very important research. An organic transistor has low charge mobility due to the nature of organic semiconductor, and thus, it cannot be used in devices requiring fast speed, which use Si, Ge, or the like. However, it can be useful in cases where elements need to be manufactured on a large area, a low process temperature or a low-priced process is required, or a bending property is particularly needed. Recently, Philips researchers reported a programmable code generator consisting of 326 transistors by using polymer for all of the substrates, electrodes, dielectric (insulator), and semiconductor, which astonished the world. This completely refutes the stereotype that a semiconductor is a hard material, and therefore, infinite applied fields thereof are foretelling depending on the human imagination.

The organic semiconductor transistor uses organic semiconductor, such as luminescent organic materials used in an organic electroluminescent transistor due to the characteristic of material, and thus, can be manufactured under the same condition as the organic electroluminescent transistor because they are same in the deposition method and similar in the physical and chemical properties. In addition, they can be manufactured by a room temperature process and a low temperature process (100°C or lower), which enables the manufacture of an organic electroluminescent device based on plastics using the organic transistor. In line with this thinking, the organic transistor can be used in a case where a liquid crystal display capable of being flexible by using plastics as substrates is realized. Meanwhile, with respect to driving of an electronic paper recently received much attention, the electronic paper employs voltage driving instead of current driving, requires high charge mobility or fast switching speed, and uses a technique applied in a large-area flexible device. Therefore, the organic transistor may be best used in the electronic paper. When the organic transistor is used in a microprocessor for a smart card being currently used based on silicon through a semiconductor process, costs accompanying the binding of silicon processor and plastic base can be saved, and thus use of the organic transistor is expected. Further, the organic transistor is thought to be applicable in various fields of computers.

In order to obtain a high-performance device, the organic semiconductor needs to satisfy general factors about charge injection and current mobility, which are as follows. (i) An organic semiconductor material needs to have such a molecular orbital (HOMO/LUMO) energy where holes and electrons can be easily injected when an electric field is applied. (ii) A crystal structure needs to have sufficient overlapping of frontier orbital so that charge movement effectively occurs between neighboring molecules. (iii) Solids need to be very pure because impurities function as charge traps. (iv) Molecules need to be selectively arranged along a long axis parallel with a device substrate so that charge movement effectively occurs along a direction of $\pi$-$\pi$ stacking within the molecules. (v) A crystal area of organic semiconductor needs to be covered in a thin film type, such as a single crystalline film between a source electrode and a drain electrode. Additively, an organic material, preferably, needs to have excellent solubility. Since a solution process is possible at a low temperature during manufacturing of device, a thin film can be formed even on a plastic substrate, thereby manufacturing the device at a low cost.

A thin film pentacene, polythiophene, polyacetylene, a-hexathienylene, fullerene (C60) or the like has been applied since a study on OTFT started in earnest from the early 1980s, and a development thereof has progressed in a direction that charge mobility and an on/off ratio, which are important characteristics of the OTFT device, can be

increased. The best p-type channel material is currently pentacene, which has a stability problem due to a change in electric characteristic caused by reaction with oxygen. The organic semiconductor is oxidized to break the bonds, thereby lowering charge mobility. In addition, lattices are distorted within crystals, and thus, charge traps occur, which causes to reduce a scattering degree and mobility of charges. In addition, many studies have been conducted that a temperature of a substrate is raised or crystallization of organic molecules is induced using a self-assembly method at the time of deposition, in order to improve the mobility of charges in the organic semiconductor. However, above all, it is important to design molecules such that inter-molecular conduction easily occurs.

Meanwhile, a solar cell is a device that directly transforms solar energy into an electric energy by applying a photovoltaic effect. A general solar cell is manufactured by p-n junction obtained by doping crystalline silicon (Si), which is an inorganic semiconductor. Electrons and holes generated due to absorption of light diffuse to a p-n junction point, and are accelerated by an electric field and moved to an electrode. A power transformation efficiency of this procedure is defined by a ratio between a power given in an outside circuit and a solar power of the solar cell, and reaches up to 24% when measured under the simulated solar irradiation conditions currently standardized. However, since the conventional inorganic solar cell already has limits in economic feasibility and available materials, an organic semiconductor solar cell, which is easily processed and cheap, and has various functions, and thus it is in the spotlight as a long-term alternative energy source.

A possibility of the organic solar cell was suggested in the 1970s, but it has no practical use due to low efficiency thereof. However, since C.W. Tang of Eastman Kodak showed a possibility of practical use as various solar cells having a double-layered structure using copper phthalocyanine (CuPc) and perylene tetracarboxylic acid derivative in 1986, an interest and a study on the organic solar cell has rapidly increased, resulting in many developments. Then, Yu., et al., introduced a bulk-heterojunction (BHC) concept in 1995, and a fullerene derivative having improved solubility, such as PCBM, was developed by using a n-type semiconductor material, thereby making a ground break in the efficiency of organic solar energy. In recent three or four years, a polymer solar cell has made a remarkable improvement in efficiency due to new constitution of elements and change of process conditions. Development of a donor material retaining a low band gap for substituting the exiting material and an acceptor material having good charge mobility is continuously being studied.

[Disclosure]

[Technical Problem]

An object of the present invention is to provide an organic semiconductor material having excellent thermal stability, solubility, and electron mobility to exhibit excellent electric characteristics, and an organic electronic device comprising the same.

[Technical Solution]

In a general aspect, there is provided an organic semiconductor material of a fullerene structure into which an aromatic fused ring compound is introduced, and more particularly, a fullerene derivative where a cyclohexane structure is introduced into fullerene, with which an aromatic ring compound or a hetero aromatic ring compound is fused, such as Chemical Formula 1 or 2 below, and an organic electronic device comprising the same.

[Chemical Formula 1]

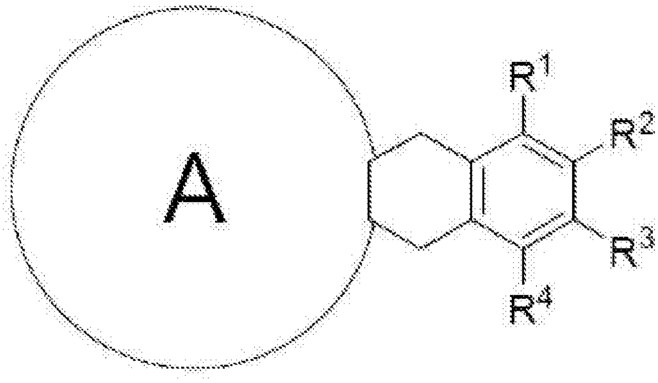

[Chemical Formula 2]

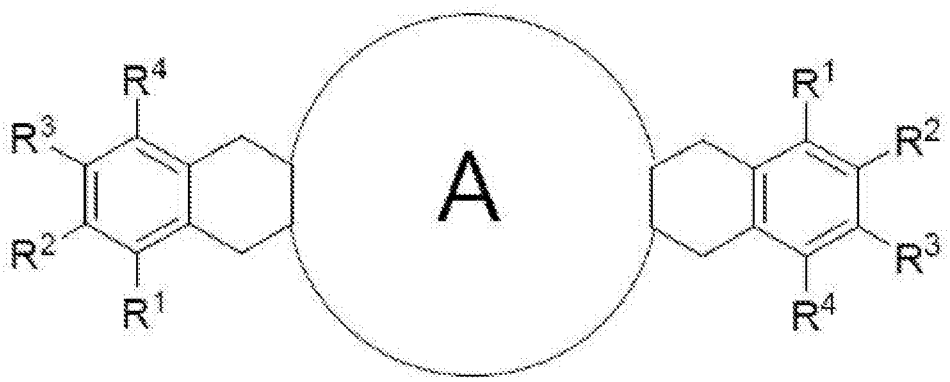

[in Chemical Formulas 1 and 2, $R^1$ through $R^4$ independently are selected from a hydrogen atom and linear or branched chain (C1-C20)alkyl, linked to an adjacent substituent via (C4-C8)alkenylene to form an aromatic fused ring, the alkenylene being substituted with one to three hetero atoms selected from an oxygen atom, a nitrogen atom, and a sulfur atom to form a hetero aromatic fused ring; A represents fullerene of C60 or C70.]

The fullerene compound according to the present invention of Chemical Formula 1 or 2, into which the aromatic fused ring compound is introduced, is specifically exemplified by the following compounds, which are not intended to limit the scope of the present invention. In addition, a position of an aromatic fused ring cyclohexane substituent of the fullerene compound is not limited in the present invention. In the fullerene derivative compound according to the present invention, a position on which an aromatic fused ring is subsituted by Diels-Alder reaction is not limited to positions which are specifically drawin in the following drawing, and may include any position of double bonds of the fullerene at which the aromatic fused ring can be substituted. Also, the fullerene derivative compound may be a mixture of position isomers. These fullerene derivative compounds have the same electrochemical properties as a fullerene derivative for an organic solar cell device.

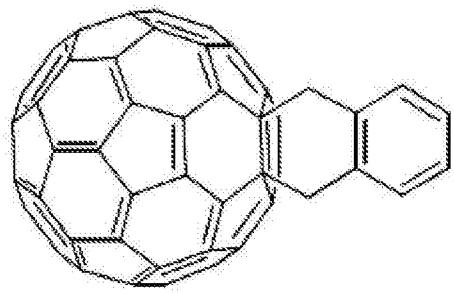

1-1

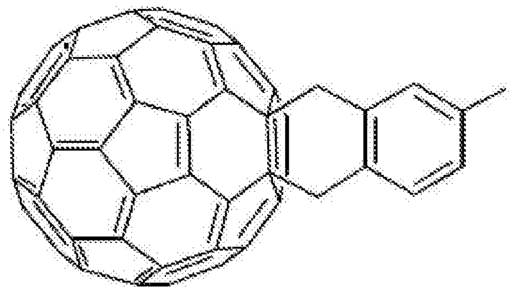

1-2

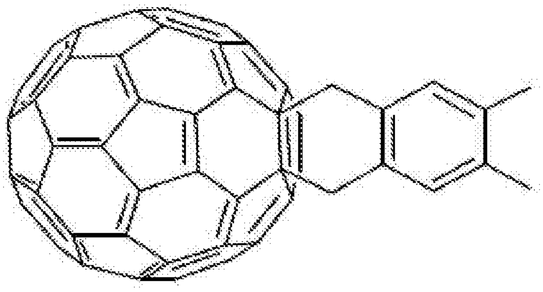

1-3

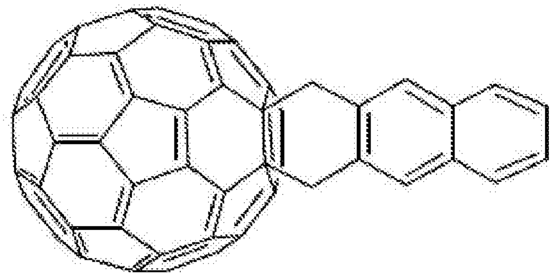

1-4

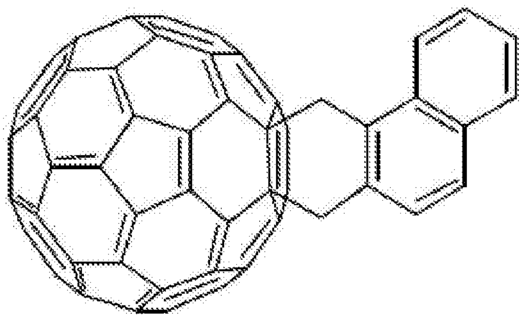

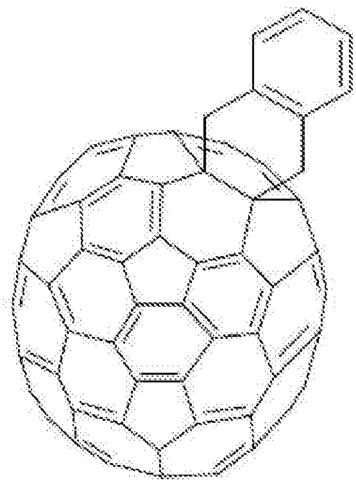

1-5 1-6

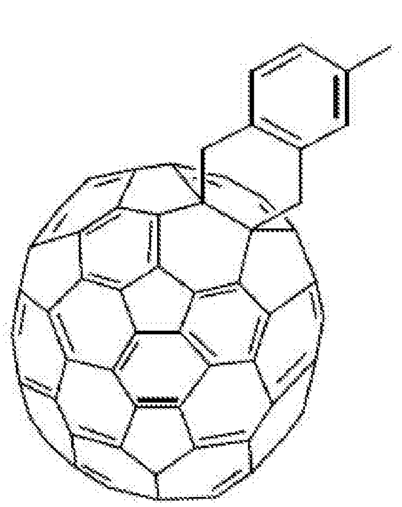

1-7

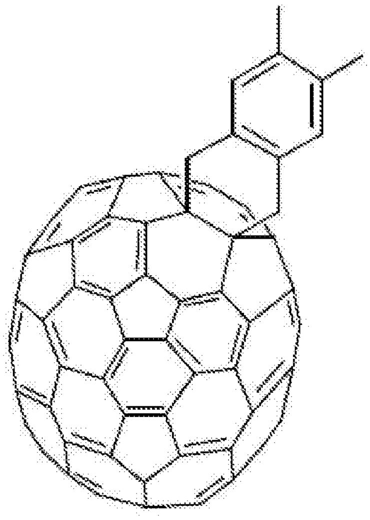

1-8

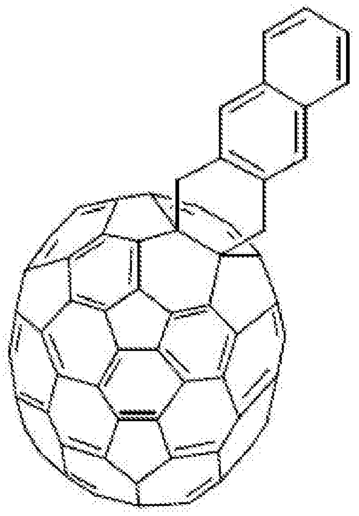

1-9

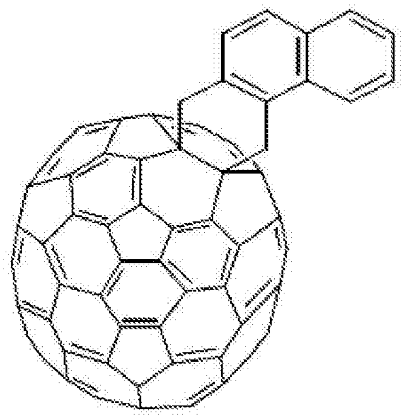

1-10

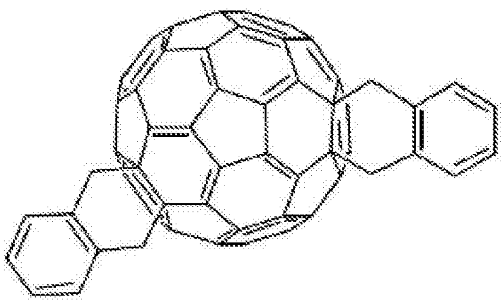

2-1

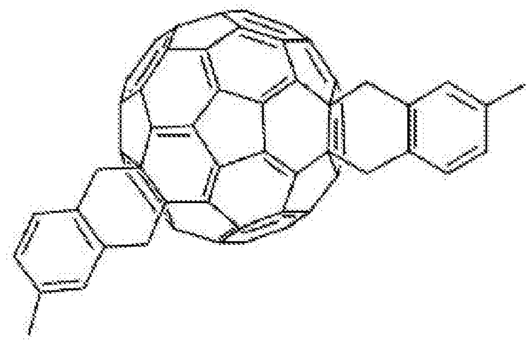

2-2

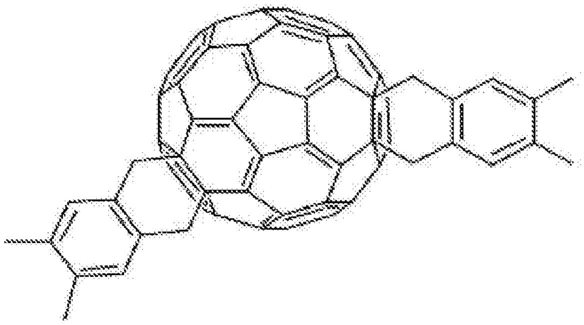

**2-3**

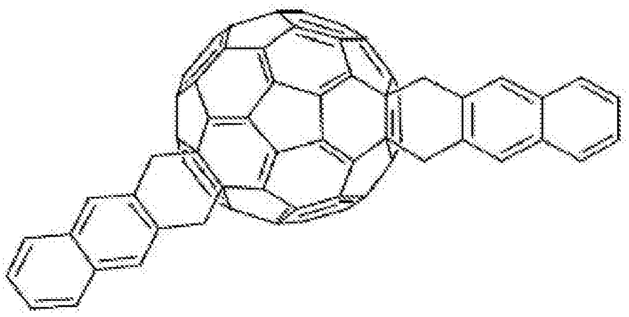

**2-4**

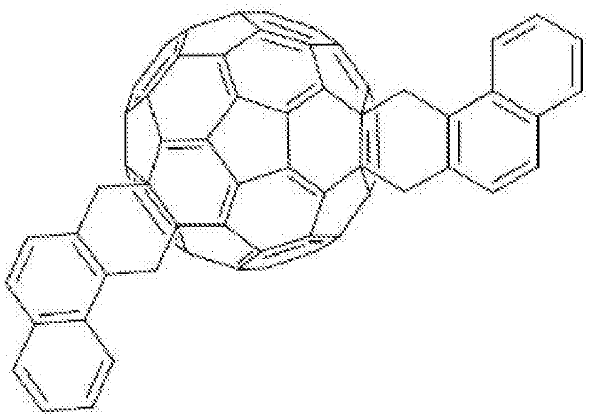

**2-5**

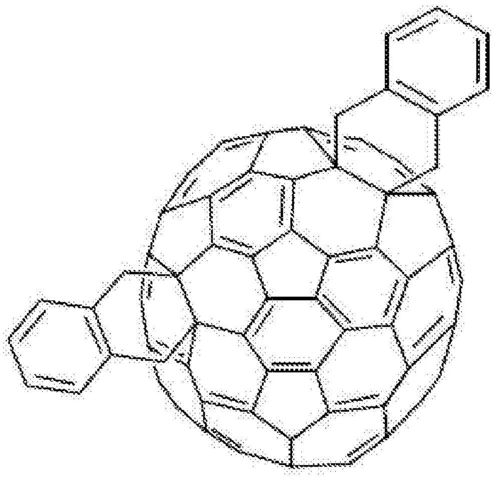

**2-6**

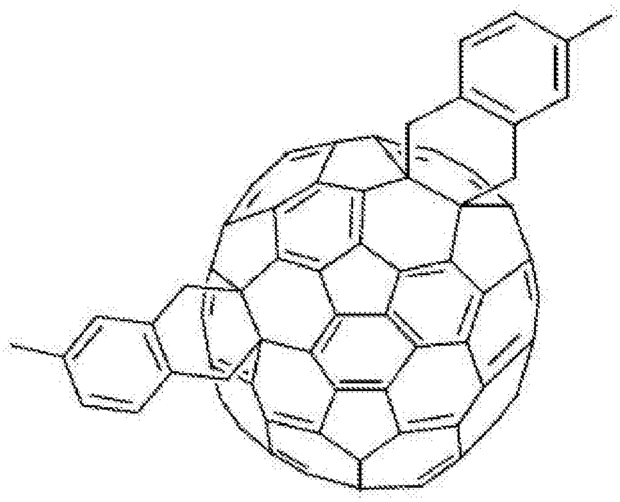

2-7

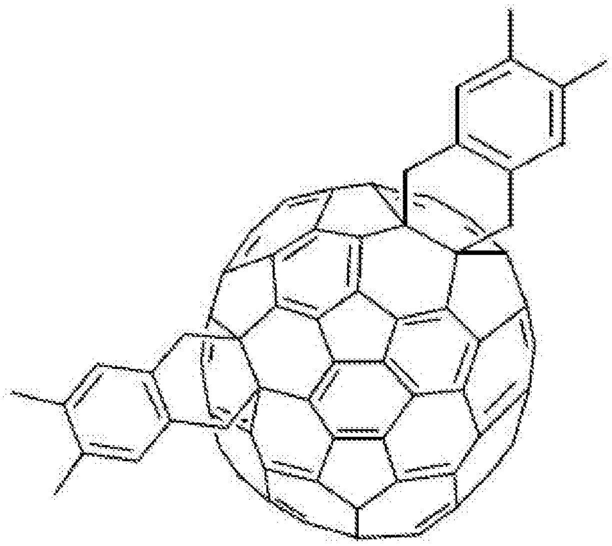

2-8

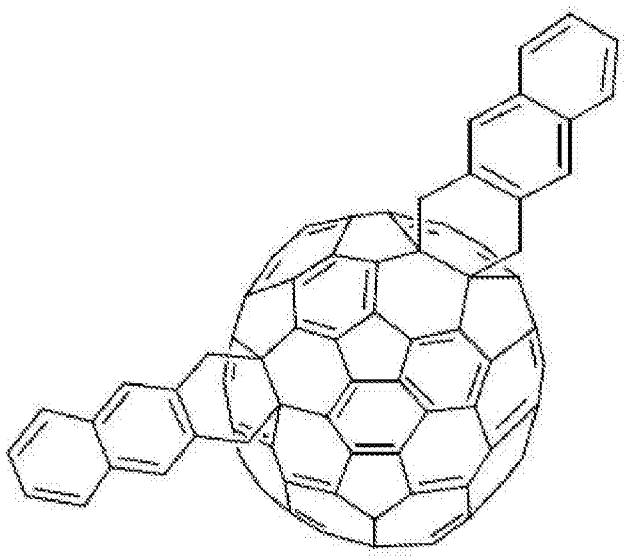

2-9

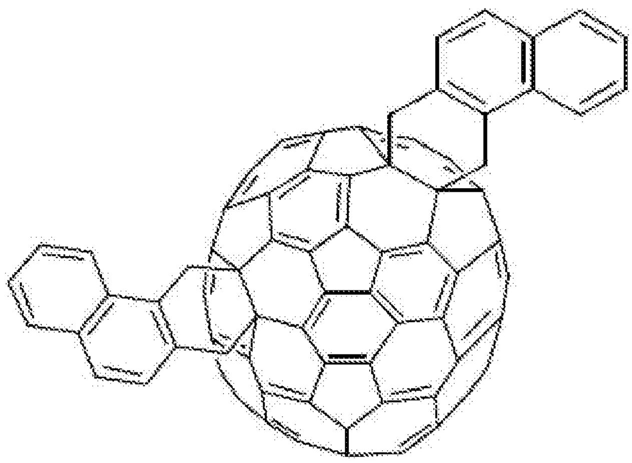

2-10

Exemplary methods of the fullerene derivative into which an aromatic fused ring compound is introduced, of Chemical Formula 1 or 2, according to the present invention, are shown in Schemes 1 to 5 below. The fullerene derivatives may be prepared by a Diels-Alder reaction, as shown in Schemes 1 to 5.The Diels-Alder reaction is an addition reaction between a diene compound having a conjugated double bond, such as butadiene, and a dienophile having a double bond or a triple bond to form a 6- or 5-membered cycle compound. The fullerene derivative of the present invention may be prepared by using any method that can use a conventional Diels-Alder reaction condition. For example, the fullerene derivative of the present invention can be obtained by heating reactants in the presence of an organic solvent, and as necessary, by further using a catalyst. Examples of the organic solvent may include aliphatic hydrocarbons, such as pentane, octane, decane, cyclohexane, and the like, aromatic hydrocarbons, such as benzene, toluene, xylene, and the like, and halogenated hydrocarbons, such as chloromethane, methylene chloride, chloroform, carbontetrachloride, 1,1-dichloroethane, 1,2-dichloethane, ethylchloride, trichloroethane, 1-chloropropane, 2-chloropropane, 1-chlorobutane, 2-chlorobutane, 1-chloro-2-methylpropane, chlorobenzene, bromobenzene, and the like.

[Scheme 1]

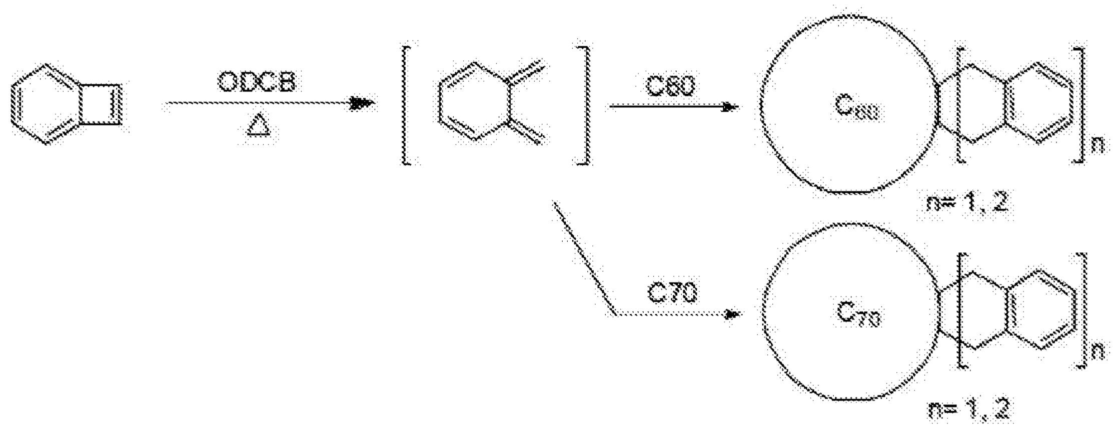


[Scheme 2]

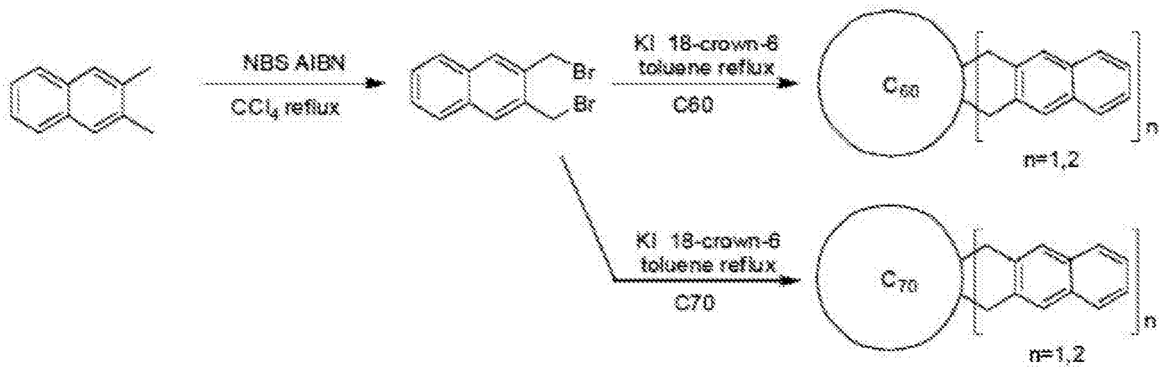

[Scheme 3]

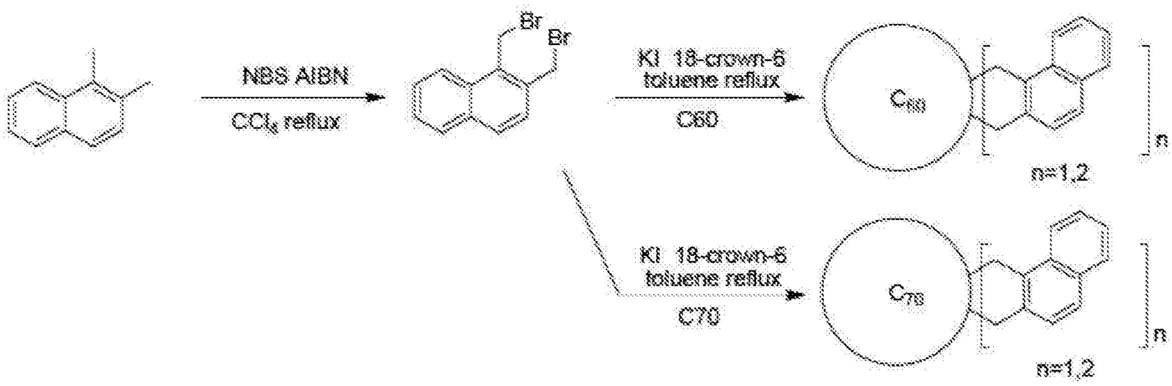


[Scheme 4]

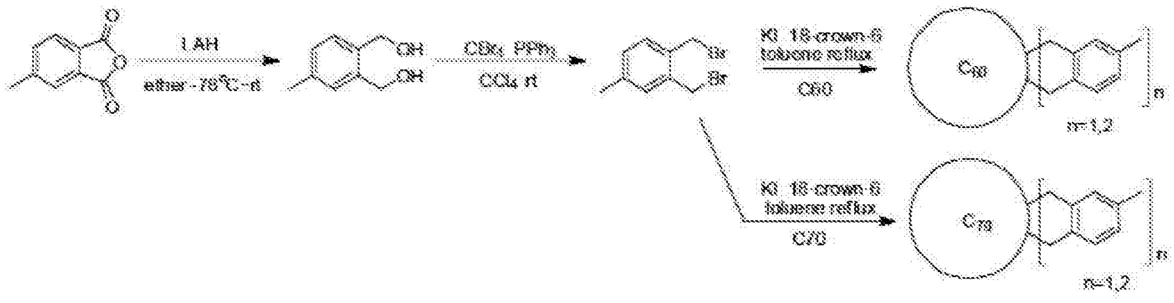


[Scheme 5]

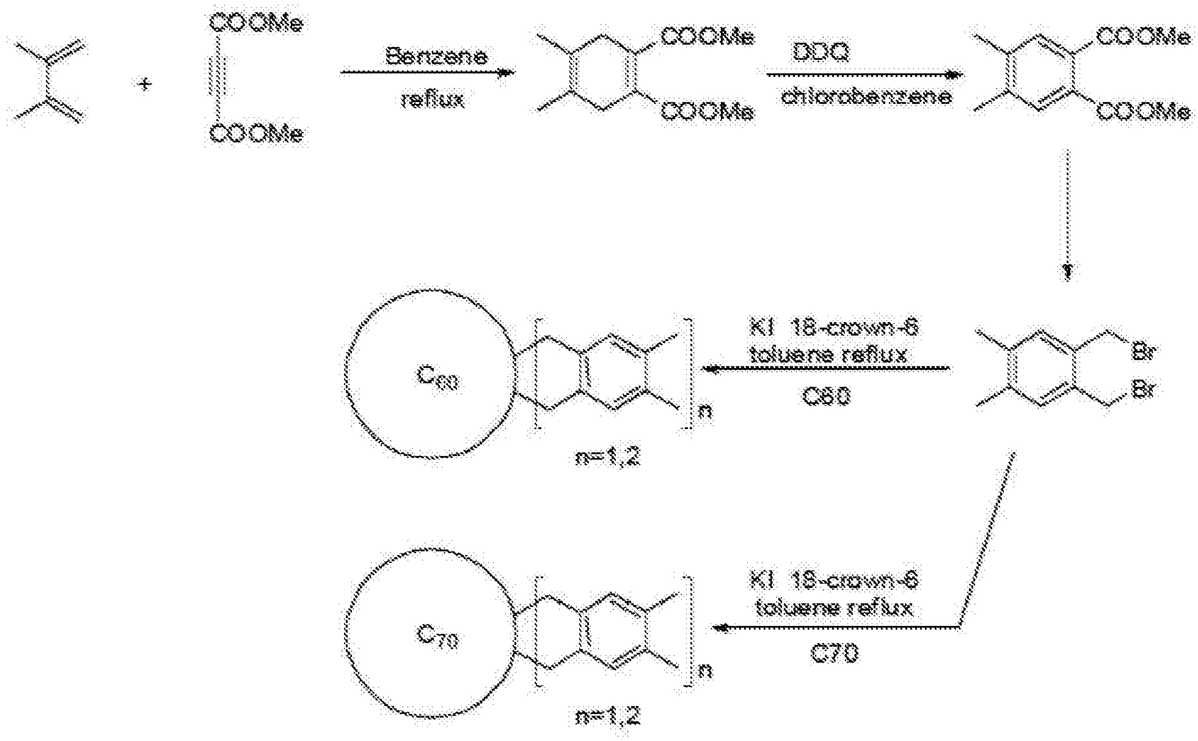


In Schemes 1 to 5, as adducts added to C60 or C70 fullerene, commercialized products may be used, or adducts added to C60 or C70 fullerene may be directly prepared. A reaction for synthesis of the fullerene and the adduct is performed in the presence of a solvent selected from the organic solvents for 6 to 48 hours while heating is performed to the boiling point of the solvent, and thus, the fullerene derivative of the present invention can be obtained.

In Schemes 1 to 5, mono-adducts and di-adducts may be simultaneously generated, and these are subjected to an ordinary separation process, such as recrystallization, column chromatography, or the like, thereby obtaining di-adducts, which are the compounds according to the present invention.

The fullerene derivative into which the aromatic fused ring compound of Chemical Formula 1 prepared by the present invention, may be used as a channel material of an organic thin film transistor, may be used for manufacturing an organic thin film transistor using the fullerene derivative of Chemical Formula 1 as a channel material, and may be used in an organic thin film transistor having an n-type organic semiconductor characteristic, which is excellent in electric mobility.

In addition, the fullerene derivative into which the aromatic fused ring compound of Chemical Formula 2 prepared by the present invention may be used in an organic solar cell device. The fullerene derivative of the present invention and an organic solar cell device using the fullerene derivative as a photoactive layer have superior electrochemical properties, as compared with the existing PCBM. The fullerene derivative has an LUMO energy level of -3.50 to -3.52 eV, which is superior by about 5%, as compared to an LUMO energy level of the existing PCBM, -3.70 eV. Therefore, it is expected that the organic solar cell device using the fullerene derivative as a photoactive layer has a higher open circuit voltage than the existing organic solar cell device using PCBM as a photoactive layer. As the result of analyzing properties of the organic solar cell device manufactured by using the fullerene derivative and regioregular poly(3-hexylthiophene) (rr-P3HT) in a photoactive layer, an open circuit voltage (Voc) of 800 to 850 mV was shown, which was further improved by 50 to 60%, as compared with PCBM. Therefore, in a case where the fullerene derivative compound of Chemical Formula 2 is used as a photoactive layer of an organic solar cell, since the fullerene derivative compound can have further improved energy conversion efficiency, and can be as a material suitable for a low-cost printing process due to excellent solubility thereof, a low-cost and high-efficiency organic solar cell device can be manufactured.

[Advantageous Effects]

In conclusion, the present invention can prepare a fullerene derivative having one cyclohexane substituent, like Chemical Formula 1, and this fullerene derivative, which is an n-type organic semiconductor material having high

solubility and excellent electron mobility, can be a channel material of an n-type organic thin film transistor through a solution process.

Furthermore, the present invention can realize a more high level of open circuit voltage (Voc) through a combination between the fullerene derivative having two cyclohexane substituents, like Chemical Formula 2, and a polymer material for a donor, thereby providing an organic solar cell device having improved power conversion efficiency.

[Description of Drawings]

FIG. 1 shows a cyclovoltametry measurement result of a fullerene compound (Compound 1-1) of Preparation example 1;
FIG. 2 shows an output curve of an organic thin film transistor device using a fullerene compound 1 (Compound 1-1) of Preparation example 1 in Example 2 as a channel material;
FIG. 3 shows a transition curve of an organic thin film transistor device using a fullerene compound 1 (Compound 1-1) of Preparation example 1 in Example 2 as a channel material;
FIG. 4 shows an output curve of an organic thin film transistor device using a fullerene compound 4 (Compound 1-4) of Preparation example 4 in Example 3 as a channel material;
FIG. 5 shows a transition curve of an organic thin film transistor device using a fullerene compound 4 (Compound 1-4) of Preparation example 4 in Example 3 as a channel material;
FIG. 6 shows an output curve of an organic thin film transistor device using PCBM of Comparative example 1 as a channel material;
FIG. 7 shows a transition curve of an organic thin film transistor device using PCBM of Comparative example 1 as a channel material;
FIG. 8 shows cyclic voltamogram of Compounds 2-1, 2-5 and 2-6 of the present invention and PCBM;
FIG. 9 shows a comparison in characteristics of organic solar cells among Examples 2 and 3 of the present invention and Comparative example 1; and
FIG. 10 shows a comparison in internal energy conversion efficiency (IPCE) measurement results of organic solar cells between Example 2 of the present invention and Comparative example 1.

[Best Mode]

Hereinafter, the present invention will be described in more detail with reference to the following exemplary embodiments. However, the following exemplary embodiments describe the present invention by way of example only but are not limited thereto.

[Preparation example 1] Preparation of Compound 1-1 and Compound 2-1

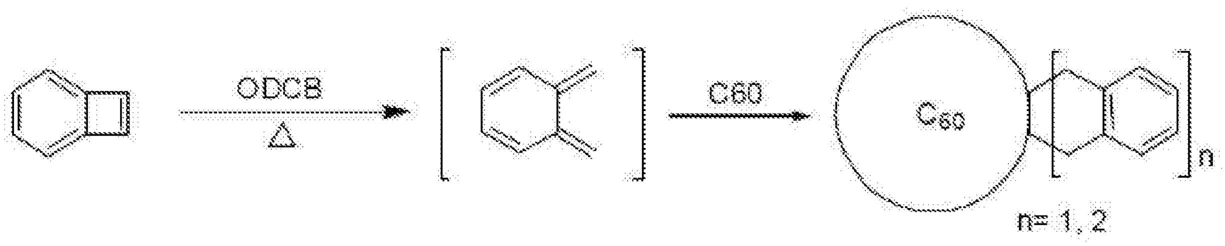


Benzocyclobutene (0.51 g, 5 mmol) and fullerene C60 (0.3 g, 0.42 mmol) were dissolved in 1,2-dichlorobenzene (50 mL) within a reaction vessel, and then reaction at 190°**C** was performed for 24 hours. After completion of the reaction, the solvent was concentrated under reduced pressure, and developed by silica gel column chromatography (40 x 10 cm) using a mixture solution of benzene and hexane (1:7), thereby obtaining brown solids, mono-adduct (Compound 1-1) (83 mg, 21%) and di-adduct (Compound 2-1) (110 mg, 28%).

Mono-adduct(Compound 1-1):

$^{1}$H-NMR 300 MHz ($CDC1_3$) δ 7.69-7.67 (m, 2H), 7.58-7.55 (m, 2H), 4.82-4.80 (m, 2H), 4.47-4.42 (m, 2H).

$^{13}$C-NMR 500 MHz ($CDCl_3$ = 77.00 ppm) δ 146.49, 146.27, 145.48, 144.74, 142.60, 142.25, 138.15, 128.02,

65.94, 45.12, 30.92.

FABMS m/z: 824 ($M^+H$): calcd. ($C_{68}H_8$), 824.

Di-adduct(Compound 2-1):

$^1H$-NMR 300 MHz ($CDCl_3$) δ 7.94-7.28 (m, 8H), 5.08-3.91 (m, 8H).

$^{13}C$-NMR 500 MHz ($CDCl_3$ = 77.00 ppm) δ 146.71, 145.41, 144.97, 144.57, 143.74, 142.43, 141.84, 141.28, 138.41, 138.05, 128.03, 127.75, 127.68, 65.06, 64.84, 64.56, 64.45, 63.79, 45.31, 45.11, 44.76, 30.92.

FABMS m/z: 928 ($M^+H$): calcd. ($C_{76}H_{16}$), 928.

[Preparation example 2] Preparation of Compound 1-2 and Compound 2-2

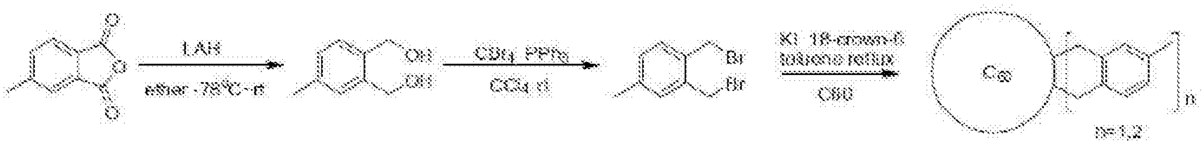


Preparation of (4-methyl-1,2-phenylene)dimethanol

4-Methylphthalic anhydride (5 g, 30.84 mmol) was dissolved in ether (90 mL), and then aluminum lithium hydride ($LiAlH_4$, LAH) (2.9 g, 77.09 mmol) was added thereinto at -78°C. The resulting mixture was stirred for 30 minutes, and then the temperature was gradually raised, followed by reaction at room temperature for 24 hours. After reaction, the resultant material was cooled by an ammonium chloride solution, and the solvent was concentrated under reduced pressure, followed by washing with ethylacetate twice and again washing with distilled water once. The organic layer is separated and then dried over sodium sulfate. Then, the solvent was concentrated under reduced pressure, and developed by silica gel column chromatography (40 x 10 cm) using a mixture solution of ethylacetate and hexane (2:5), thereby obtaining white solids, (4-methyl-1,2-phenylene)dimethanol (3.73 g, 80%).

$^1H$-NMR 300 MHz ($CDCl_3$) δ 7.21-7.11 (m, 3H), 4.62 (s, 4H), 3.48 (brs, 1H), 3.40 (brs, 1H), 2.34 (s, 3H)

Preparation of 1,2-bis(bromomethyl)-4-methylbenzene

(4-methyl-1,2-phenylene)dimethanol (3 g, 19.71 mmol), tetrabromomethane (13.08 g, 39.42 mmol), and triphenylphosphine (10.34 g, 39.42 mmol) were dissolved in tetrachloromethane (150 mL), and then reaction at room temperature was performed for 24 hours. After the reaction, the solvent was concentrated under reduced pressure, followed by washing with ethylacetate twice and again washing with distilled water once. The organic layer was separated, and then dried over sodium sulfate. Then, the solvent was concentrated under reduced pressure, and developed by silica gel column chromatography (40 x 10 cm) using a mixture solution of ethylacetate and hexane (2:5), thereby obtaining white solids, 1,2-bis(bromomethyl)-4-methylbenzene (1.44 g, 26%).

Preparation of C60 derivative using Diels-Alder reaction

1,2-bis(bromomethyl)-4-methylbenzene (0.76 g, 2.76 mmol), potassium iodide (KI, 0.69 g, 4.17 mmol), 18-crown-6 (1.82 g, 6.9 mmol), and fullerene C60 (0.5 g, 0.69 mmol) were dissolved in toluene (100 mL), and reaction under reflux at 110°**C** was performed for 24 hours. After the reaction, the solvent was concentrated under reduced pressure, followed by washing with dichloromethane twice and again washing with distilled water once. The organic layer was separated, and then dried over sodium sulfate. The solvent was concentrated under reduced pressure, and developed by silica gel column chromatography (40 x 10 cm) using a mixture solution of benzene and hexane (2:7), thereby obtaining brown solids, mono-adduct (Compound 1-2) (10 mg) and di-adduct (Compound 2-2) (7 mg).

Mono-adduct (Compound 1-2):

$^1H$-NMR 300 MHz ($CDCl_3$) δ 7.57-7.55 (m, 1H), 7.50 (s, 1H), 7.37-7.35 (m, 1H), 4.81-4.77 (m, 2H), 4.42-4.38 (m, 2H), 2.55 (s, 3H).

FABMS m/z: 839 ($M^+$+1); calcd. ($C_{69}H_{10}$) 838.

Di-adduct(Compound 2-2):

$^1H$-NMR 300 MHz ($CDCl_3$) δ 7.59-7.32 (m, 2H), 7.52 (s, 2H), 7.41-7.33 (m, 2H), 4.81-4.77 (m, 4H), 4.42-4.38 (m, 4H), 2.55 (s, 6H).

FABMS m/z: 957 ($M^{+}$+1); calcd. ($C_{78}H_{20}$) 956.

[Preparation example 3] Preparation of Compound 1-3 and Compound 2-3

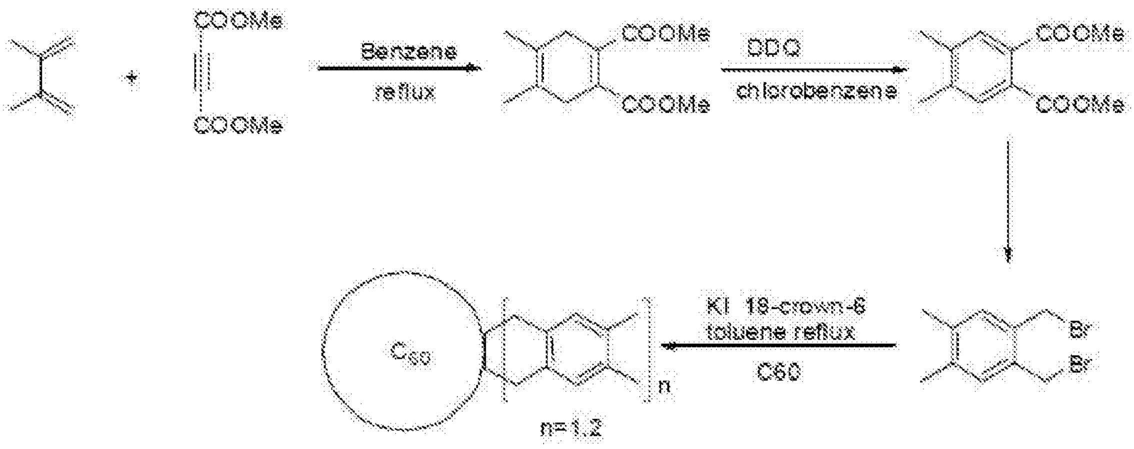


Preparation of dimethyl 4,5-dimethylcyclohexa-1,4-diene-1,2-dicarboxylate

Dimethyl acetylendicarboxylate (5 g, 35.18 mmol) was dissolved in benzene (50 mL), and then 2,3-dimethyl-1,3-butadiene (2.72 g, 33.07 mmol) was added thereinto in the presence of nitrogen, followed by stirring under reflux for 24 hours. After the reaction, the solvent was concentrated under reduced pressure, followed by washing with ethylacetate twice and again washing with distilled water once. The organic layer was separated, and then dried over sodium sulfate. Then, the solvent was concentrated under reduced pressure, and developed by silica gel column chromatography (40 x 10 cm) using a mixture solution of ethylacetate and hexane (1:5), thereby obtaining white solids, 4,5-dimethylcyclohexa-1,4-diene-1,2-dicarboxylate (5.68 g, 72%).

$^{1}$H-NMR 300 MHz ($CDCl_3$) $\delta$ 3.77 (s, 6H), 2.92 (s, 4H), 1.66 (s, 6H)

Preparation of dimethyl 4,5-Dimethylbenzene-1,2-dicarboxylate

Dimethyl 4,5-dimethylcyclohexa-1,4-diene-1,2-dicarboxylate (2 g, 8.92 mmol) was dissolved in Chlorobenzene (50 mL), and then 2,3-dichloro-5,6-dicyano-1,4-benzoquinone (DDQ) (4 g, 17.84 mmol) was slowly added thereinto little by little at room temperature, followed by stirring under reflux for 24 hours. After the reaction, the solvent was concentrated under reduced pressure, followed by washing with ethylacetate twice and again washing with distilled water once. The organic layer was separated, and then dried over sodium sulfate. Then, the solvent was concentrated under reduced pressure, and developed by silica gel column chromatography (40 x 10 cm) using a mixture solution of ethylacetate and hexane (1:5), thereby obtaining transparent oil, dimethyl 4,5-dimethylbenzene-1,2-dicarboxylate (1.29 g, 65%).

$^{1}$H-NMR 300 MHz ($CDCl_3$) $\delta$ 7.49 (s, 2H), 3.88 (s, 6H), 2.31 (s, 6H)

Preparation of 1,2-bis(bromomethyl)-4,5-dimethylbenzene

Dried tetrahydrofurane (20 mL) was put into LAH (0.54 g, 14.29 mmol) at -78°C. Dimethyl 4,5-dimethylbenzene-1,2-dicarboxylate (1.27 g, 5.71 mmol) was dissolved in dried tetrahydrofurane (10 mL), and then the resultant mixture was slowly added into the above reaction solution. Then the reaction temperature was slowly raised to room temperature, and then stirring under reflux was performed for 24 hours. After the reaction, the resultant reaction material was cooled by a sodium hydroxide solution, and then concentrated under reduced pressure, followed by washing with ethyl acetate twice and again washing with distilled water once. The organic layer was separated, and dried over sodium sulfate, and then the solvent was concentrated under reduced pressure, thereby obtaining white solids, (4,5-dimethyl-1,2-phenylene) dimethanol (0.95 g, quantitative). This is subjected to a bromination reaction using tribromophosphine, thereby obtaining 1,2-bis(bromomethyl)-4,5-dimethyl benzene at a yield of 56%.

$^1$H-NMR 300 MHz ($CDCl_3$) δ 7.13 (s, 2H), 4.27 (s, 4H), 2.23 (s, 6H).

Preparation of C60 derivative using Diels-Alder reaction

The same method as Preparation example 2 was performed by using 1,2-bis(bromomethyl)-4,5-dimethylbenzene (0.6 g, 2.06 mmol), potassium iodide (KI, 0.69 g, 4.17 mmol), 18-crown-6 (1.82 g, 6.9 mmol), fullerene 60 (Fullerene C60, 0.5 g, 0.69 mmol), to obtain brown solids, mono-adduct (Compound 1-3) (9 mg) and di-adduct (Compound 2-3) (5 mg).

mono-adduct(Compound 1-3):

$^1$H-NMR 300 MHz ($CDCl_3$) δ 7.54 (s, 2H), 4.52-4.39 (m, 4H), 2.54 (s, 6H).

FABMS m/z: 852 ($M^+$); calcd. ($C_{70}H_{12}$), 852.

Di-adduct(Compound 2-3):

$^1$H-NMR 300 MHz ($CDCl_3$) δ 7.59-7.52 (m, 4H), 4.55-4.36 (m, 8H), 2.64-2.51 (m, 6H).

FABMS m/z: 984 ($M^+$); calcd. ($C_{78}H_{20}$), 984.

[Preparation example 4] Preparation of Compound 1-4 and Compound 2-4

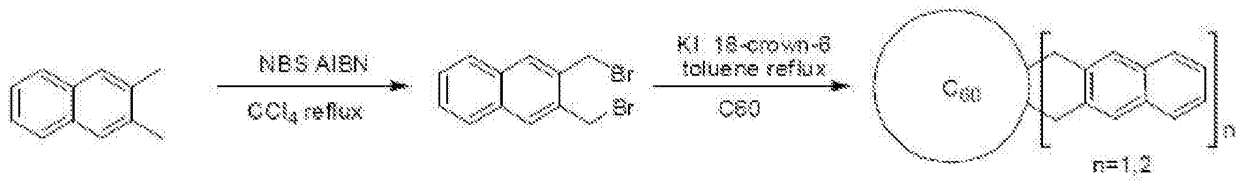


Preparation of 2,3-bis(bromomethyl)naphthalene

2,3-Dimethylnaphthalene (3 g, 19.2 mmol), N-bromosuccimide (6.84 g, 38.4 mmol), and 2,2'-azobis(2-methylpropionitrile (AIBN, 321 mg, 0.19 mmol) were dissolved in carbon tetrachloride (60 mL), and then reaction under reflux at 80°C was performed for 24 hours. After completion of the reaction, the solvent was concentrated under reduced pressure and then recrystallized by hexane, thereby obtaining beige solids, 2,3-bis(bromomethyl)naphthalene (4.47 g, 74%).

$^1$H-NMR 300 MHz ($CDCl_3$) δ 7.86 (s, 2H), 7.81-7.78 (m, 2H), 7.52-7.49 (m, 2H), 4.87 (s, 4H)

Preparation of C60 derivative using Diels-Alder reaction

The same method as Preparation example 2 was performed by using 2,3-bis(bromomethyl)naphthalene (1.3 g, 4.17 mmol), potassium iodide (KI, 0.69 g, 4.17 mmol), 18-crown-6 (1.82 g, 6.9 mmol), fullerene 60 (Fullerene C60, 0.5 g, 0.69 mmol), to obtain brown solids, mono-adduct (Compound 1-4) (140 mg, 20%) and di-adduct (Compound 2-4) (85mg, 10%).

Mono-adduct (Compound 1-4):

$^1$H-NMR 300 MHz ($CDCl_3$) δ 7.83-7.77 (m, 4H), 7.25-7.47 (m, 2H), 4.88-4.83 (m, 4H).

FABMS m/z: 874 ($M^+$H): calcd. ($C_{72}H_{10}$), 874.

Di-adduct (Compound 2-4):

$^1$H-NMR 300 MHz ($CDCl_3$) δ 7.89-7.70 (m, 8H), 7.53-7.17 (m, 4H), 4.88-4.79 (m, 8H).

FABMS m/z: 1028 ($M^+$H): calcd. ($C_{84}H_{20}$), 1028.

[Preparation example 5] Preparation of Compound 1-5 and Compound 2-5

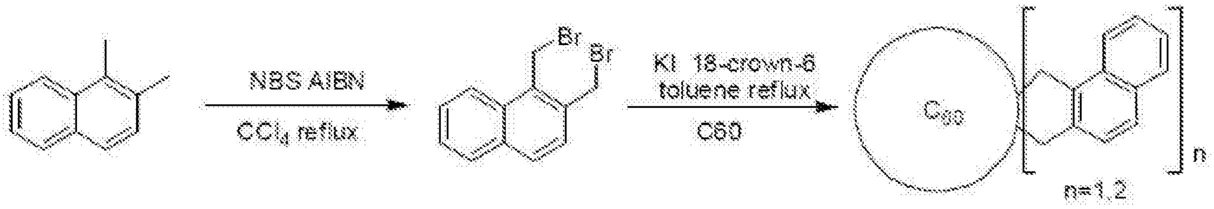


Preparation of 1,2-bis(bromomethyl)naphthalene

The same method as Preparation example 1 was performed by using 1,2-dimethylnaphthalene (2 g, 12.8 mmol), N-bromosuccimide (4.56 g, 25.6 mmol), and 2,2'-azobis(2-methylpropionitrile (AIBN, 11 mg, 0.064 mmol), to obtain 1,2-bis(bromomethyl)naphthalene (3.5 g, 87%).
$^1$H-NMR 300 MHz ($CDCl_3$) δ 8.13 (d, J = 8.4 Hz, 1H), 7.83 (t, J = 8.4 Hz, 2H), 7.66-7.60 (m, 1H), 7.55-7.50 (m, 1H), 7.42 (d, J = 8.4 Hz, 1H), 5.09 (s, 2H), 4.76 (s, 2H)

Preparation of C60 derivative using Diels-Alder reaction

The same method as Preparation example 2 was performed by using 1,2-bis(bromomethyl)naphthalene (0.87 g, 2.76 mmol), potassium iodide (KI, 0.69 g, 4.17 mmol), 18-crown-6 (1.82 g, 6.9 mmol), fullerene C60 (0.5 g, 0.69 mmol), to obtain brown solids, mono-adduct (Compound 1-5) (102 mg, 14%) and di-adduct (Compound 2-5) (68 mg).
Mono-adduct (Compound 1-5):
$^1$H-NMR 300 MHz ($CDCl_3$) δ 8.61-7.52 (m, 6H), 5.25-4.12 (m, 4H).
FABMS m/z: 874 ($M^+$H): calcd. ($C_{72}H_{10}$), 874.
Di-adduct (Compound 2-5):
$^1$H-NMR 300 MHz ($CDCl_3$) δ 8.75-7.48 (m, 12H), 5.29-4.01 (m, 8H).
FABMS m/z: 1028 ($M^+$H): calcd. ($C_{84}H_{20}$), 1028.

[Preparation example 6] Preparation of Compound 1-6 and Compound 2-6

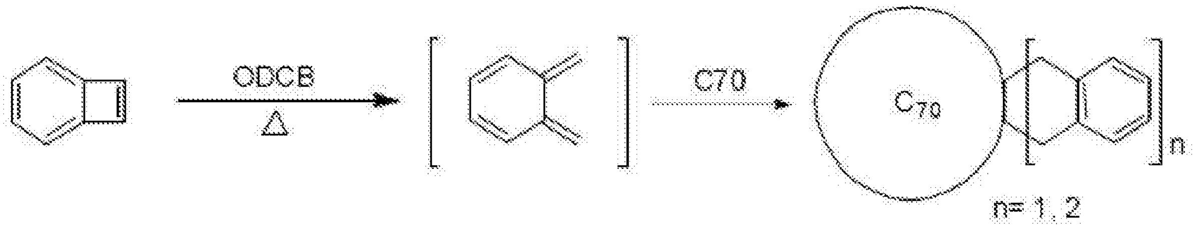


The same method as Preparation example 1, except that fullerene C70 (0.5 g, 0.69 mmol) was used instead of fullerene C60, was performed to obtain brown solids, mono-adduct (Compound 1-6) (112 mg, 18%) and di-adduct (Compound 2-6) (220 mg, 35%).
Mono-adduct (Compound 1-6):
$^1$H-NMR 300 MHz ($CDCl_3$) δ 7.70-7.67 (m, 2H), 7.57-7.53 (m, 2H), 4.83-4.79 (m, 2H), 4.47-4.41 (m, 2H).
FABMS m/z: 1048 ($M^+$H): calcd. ($C_{86}H_{16}$), 1048.
Di-adduct (Compound 2-6):
$^1$H-NMR 300 MHz ($CDCl_3$) δ 7.58-7.36 (m, 8H), 4.18-3.66 (m, 8H).
FABMS m/z: 1048 ($M^+$H): calcd. ($C_{86}H_{16}$), 1048.

[Preparation example 7] Preparation of Compound 1-7 and Compound 2-7

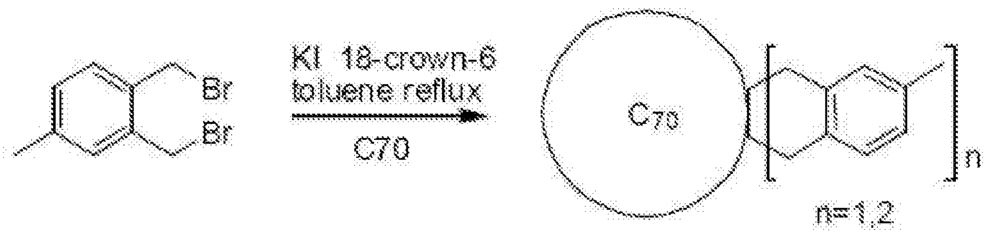


The same method as Preparation example 2, except that fullerene C70 (0.58g, 0.69 mmol) was used instead of fullerene C60, was performed to obtain brown solids, mono-adduct (Compound 1-7) (23 mg) and di-adduct (Compound 2-7) (15 mg).

mono-adduct (Compound 1-7):

$^{1}$H-NMR 300 MHz ($CDCl_3$) δ 7.53-7.50 (m, 1H), 7.47 (s, 1H), 7.32-7.30 (m, 1H), 4.80-4.76 (m, 2H), 4.42-4.39 (m, 2H), 2.53 (s, 3H).

FABMS m/z: 958 ($M^+$); calcd. ($C_{79}H_{10}$) 958.

Di-adduct (Compound 2-7):

$^{1}$H-NMR 300 MHz ($CDCl_3$) δ 7.60-7.29 (m, 2H), 7.55-7.47 (m, 2H), 7.41-7.30 (m, 2H), 4.83-4.72 (m, 4H), 4.45-4.33 (m, 4H), 2.59-2.44 (m, 6H).

FABMS m/z: 1076 ($M^+$); calcd. ($C_{88}H_{20}$) 1076.

[Preparation example 8] Preparation of Compound 1-8 and Compound 2-8

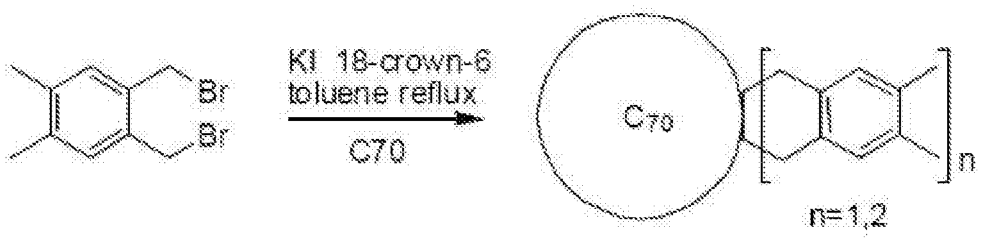


The same method as Preparation example 3, except that fullerene C70 (0.58g, 0.69 mmol) was used instead of fullerene C60, was performed to obtain brown solids, mono-adduct (Compound 1-8) (19 mg) and di-adduct (Compound 2-8) (25 mg).

mono-adduct (Compound 1-8):

$^{1}$H-NMR 300 MHz ($CDCl_3$) δ 7.51 (s, 2H), 4.50-4.39 (m, 4H), 2.55 (s, 6H).

FABMS m/z: 972 ($M^+$); calcd. ($C_{80}H_{12}$), 972.

Di-adduct (Compound 2-8):

$^{1}$H-NMR 300 MHz ($CDCl_3$) δ 7.57-7.47 (m, 4H), 4.59-4.29 (m, 8H), 2.61-2.57 (m, 6H).

FABMS m/z: 1104 ($M^+$); calcd. ($C_{90}H_{24}$), 1104.

[Preparation example 9] Preparation of Compound 1-9 and Compound 2-9

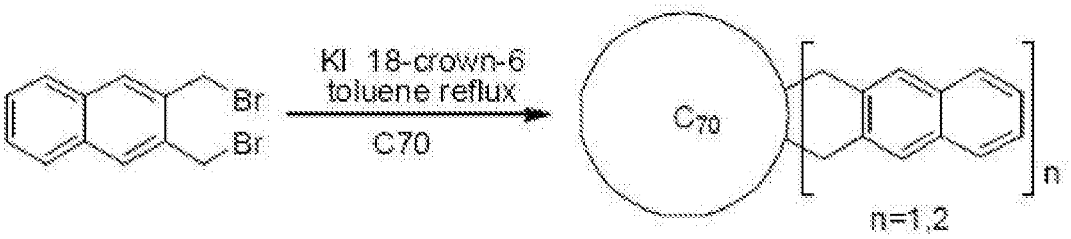


The same method as Preparation example 4, except that fullerene C70 (0.58g, 0.69 mmol) was used instead of fullerene C60, was performed to obtain brown solids, mono-adduct (Compound 1-9) (85 mg, 12%) and di-adduct (Compound 2-9) (168mg, 21%).

Mono-adduct (Compound 1-9):

$^1$H-NMR 300 MHz ($CDCl_3$) δ 7.87-7.79 (m, 4H), 7.49-7.27 (m, 2H), 4.91-4.80 (m, 4H).

FABMS m/z: 994 ($M^+$H): calcd. ($C_{82}H_{10}$), 994.

Di-adduct (Compound 2-9):

$^1$H-NMR 300 MHz ($CDCl_3$) δ 7.93-7.65 (m, 8H), 7.59-7.10 (m, 4H), 4.91-4.70 (m, 8H).

FABMS m/z: 1148 ($M^+$H): calcd. ($C_{84}H_{20}$), 1148.

[Preparation example 10] Preparation of Compound 1-10 and Compound 2-10

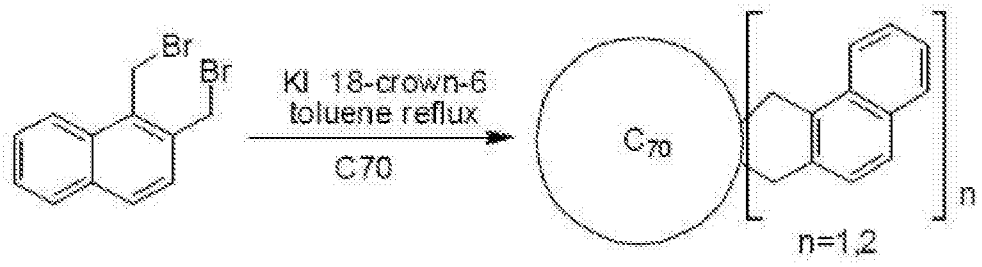


The same method as Preparation example 4, except that fullerene C70 (0.58 g, 0.69 mmol) was used instead of fullerene C60, was performed to obtain brown solids, mono-adduct (Compound 1-10) (77 mg, 11%) and di-adduct (Compound 2-10) (192 mg, 24%).

Mono-adduct (Compound 1-10):

$^1$H-NMR 300 MHz ($CDCl_3$) δ 8.57-7.47 (m, 6H), 5.21-4.10 (m, 4H).

FABMS m/z: 994 ($M^+$H): calcd. ($C_{82}H_{10}$), 994.

Di-adduct (Compound 2-10):

$^1$H-NMR 300 MHz ($CDCl_3$) δ 8.79-7.43 (m, 12H), 5.31-4.00 (m, 8H).

FABMS m/z: 1148 ($M^+$H): calcd. ($C_{84}H_{20}$), 1148.

[Example 1] Electrochemical properties of fullerene derivative compound

Oxidation/reduction characteristics using a Cyclovoltameter(CV) were measured in order to determine electrochemical properties of the fullerene compound (Compound 1-1) prepared in Preparation example 1. A BAS 100 cyclovoltametry was used as the CV equipment, 0.1M solvent of tetrabutylammonium tetrafluoroborate ($Bu_4NBF_4$) and acetonitrile was used as an electrolyte, and $10^{-3}$ M of a specimen was dissolved in 1,2-dichlorobenzene. Measurement was performed at a scan rate of 100 mW/s, at room temperature under argon. A glass carbon electrode (diameter 0.3 mm) was used as a working electrode, and a palladium(Pt) electrode and a silver/silver chloride (Ag/AgCl) electrode were used as a counter electrode and a reference electrode. The results were shown in FIG. 1.

[Example 2] Manufacture and measurement of organic thin film transistor device comprising fullerene derivative compound (Compound 1-1) of Preparation example 1

An organic thin film transistor device was manufactured by using a fullerene compound (Compound 1-1) obtained through the reaction with benzocylcobutene of Preparation example 1 among fullerene derivatives. The device was manufactured as follows. 300 nm of silicon wafer was sulfuric acid-treated with a piranha solution of sulfuric acid and hydrogen peroxide (4:1) on a hot plate at 100**°C** for 20 minutes. The sulfuric acid and hydrogen peroxide on the sulfuric acid-treated silicon wafer wiped off by using distilled water several times, and then moisture on a surface of the silicon wafer was removed while blowing nitrogen. The surface of the silicon wafer after all treatments was UV/ozone-treated for 20 minutes, and hexamethyldisilane (HMDS)-treated by using a spin coating method (0 rpm, 30 s, 4000 rpm, 30s).

After surface treatment was finished, heat treatment was performed on the resultant silicon wafer at 120**°C** for 10 minutes. After the heat treatment was finished, a solution in which the fullerene compound of Preparation example 1 was dissolved chlorobenzene in a concentration of 1 wt% was spin-coated on the resultant silicon wafer (500 rpm, 5 s, 2000 rpm, 60 s). Here, the thickness of an organic material was 30 nm, as the measurement result by an Alpha step.

After spin coating, baking was performed at 90**°C** under the condition of nitrogen air current within a glove box for 20 minutes. After baking, a source and a drain was formed by deposition. Herein, a base pressure was $10^{-6}$ torr, and the source and the drain were formed by deposition of aluminum having a work function of 4.2 eV (120 nm). Here, when aluminum was used to form the source and the drain, magnesium (Mg, 5 nm) was deposited in order to prevent oxidation of metal. The material may be oxidized at the time of measurement. Therefore, a glass cap with a getter was attached on a channel by using epoxy, and thus, absorption of moisture can be prevented UV curing for 90 seconds was performed to finish the manufacture. After all working processes were finished, silver painting was performed at room temperature and a gate was attached, and then electron mobility characteristic was evaluated.

As can be seen from FIGS. 2 and 3, the results confirmed an on/off ratio of $10^5$ or higher, an excellent transition curve according to the change of gate voltage of 0 to 40V, and excellent electron mobility of 0.0387 $cm^2/Vs$.

[Example 3] Manufacture and measurement of organic thin film transistor device comprising fullerene derivative compound of Preparation example 4

An organic thin film transistor device was manufactured by the same method as Example 2, except that the fullerene derivative compound (Compound 1-4) of Preparation example 4 was used as a channel material, and an electron mobility characteristic thereof was evaluated.

As can be seen from FIGS. 4 and 5, the results confirmed an on/off ratio of $10^5$ or higher, an excellent transition curve according to the change of gate voltage of 0 to 40 V, and excellent electron mobility of 0.0101 $cm^2/Vs$.

[Comparative example 1] Manufacture of OTFT device using PCBM as a channel material

An OTFT device was manufactured by the same method as Example 2, except that PCBM was used as a channel material, and an electron mobility characteristic thereof was evaluated.

As can be seen from FIGS. 6 and 7, the results confirmed an on/off ratio of about $10^4$, an excellent transition curve according to the change of gate voltage of 0 to 40 V, and excellent electron mobility of 0.0058 $cm^2/Vs$.

Table 1 shows comparison in characteristics of the OTFT devices manufactured in Examples 2 and 3 and Comparative example 1.

[Table 1] Comparison of the OTFT performance among the existing PCBM and the fullerene compounds of the present invention

| Device | Compound | Film forming method | S/D electrode | Electron mobility ($cm^2/Vs$) | On/off ratio $I_{on}/I_{off}$ | Threshold voltage ($V_{th}$) |
|---|---|---|---|---|---|---|
| Example 2 | Compound 1-1 | Spin coating | Mg/Al | 0.0387 | $10^5$ | 16.84 |
| Example 3 | Compound 1-4 | Spin coating | Mg/Al | 0.0101 | $10^5$ | 17.69 |
| Comparative example 1 | PCBM | Spin coating | Mg/Al | 0.0058 | $10^4$ | 17.90 |

The results showed that each case where Compounds 1-1 and 1-4 of Examples 2 and 3 of the present invention were used as a channel material has a higher on/off ratio and superior electron mobility, as compared with a case where the existing PCBM (Comparative example 1) was used as a channel material. In particular, the device of Example 2 (Compound 1-1 of Preparation example 1) showed very good electron mobility of 0.0387 $cm^2/Vs$, which is six times higher than 0.0058 $cm^2/Vs$ obtained in a case where PCBM, the existing representative fullerene derivative, was used. In addition, the devices of Examples 2 and 3 had an excellent on/off ratio of $10^5$ or higher, considering that the device using the existing PCBM was $10^4$.

[Example 4] Electrochemical properties of fullerene derivative compound

Oxidation/reduction characteristics using a Cyclovoltameter(CV) were measured in order to determine electrochemical properties of the fullerene compounds prepared in Preparation example 1 (Compound 2-1), Preparation example 5 (Compound 2-5), and Preparation example 6 (Compound 2-6). A BAS 100 cyclovoltametry was used as the CV equipment, 0.1M solvent of tetrabutylammonium tetrafluoroborate ($BU_4NBF_4$) and acetonitrile was used as an electrolyte, and $10^{-3}$ M of a specimen was dissolved in 1,2-dichlorobenzene. Measurement was performed at a scan rate of 100 mW/s, at room temperature under argon. A glass carbon electrode (diameter 0.3 mm) was used as a working electrode, and a Pt electrode and a Ag/AgCl electrode were used as a counter electrode and a reference electrode. The results were shown in FIG. 8 and Table 2.

[Table 2] Electrochemical properties of fullerene compound including aromatic fused ring

| Compound | $E^1_{1/2}$ (V)[a] | $E^2_{1/2}$ (V) | [a] LUMO (eV)[b] |
|---|---|---|---|
| PCBM | - | - | -3.70 |
| Compound 2-1 | -1.087 | -1.473 | -3.46 |
| Compound 2-6 | -1.065 | -1.411 | -3.48 |
| [a] Half wave potential was obtained by using a ferrocene standard material under 0.1M solution of $Bu_4NPF_6$ and $CH_2Cl_2$.<br>[b] Value calculated by defining the energy level of ferrocene as -4.8 eV. | | | |

In general, it has been known that an open circuit voltage (Voc) of an organic solar cell is due to a difference between an HOMO energy level of a donor material and an LUMO energy level of an acceptor material (C.J. Brabec et al, Adv. Func. Mater., 2001, 11, 374).As shown in Table 2, the fullerene compounds including an aromatic fused ring of the present invention have an LUMO energy level, which is higher by 0.18 to 0.20 eV as compared with the existing PCBM, and thus, can provide a higher open circuit voltage to an organic solar cell device.

[Example 5] Organic solar cell device using P3HT and Compound 2-1 as photoactive layer

After PEDOT-PSS (Bayer Baytron P, Al 4083) was spin-coated with the thickness of 40nm on the washed indium tin oxide (ITO) glass substrate (sheet resistance 7 Ω/sq), poly-3-(hexylthiophene) (P3HT, Rieke Metal Company) and a fullerene derivative including the aromatic fused ring (Compound 2-1) prepared in the present invention was dissolved in 1,2-dichlororbenzne, chlorobenzne, or chloroform alone or a mixture thereof. Then spin coating using the resultant solution was performed to form an organic thin film. On the organic layer thus obtained, LiF/Al were deposited under vacuum to form electrodes in 0.7 nm and 120 nm, respectively, which were then sealed by a glass cap with absorbent. The sealed device was annealed at 150°**C** for 10 minutes, and I-V characteristic thereof was measured by using a class A solar simulator (Newport Company) under a light source of AM 1.5G 100 $mW/cm^2$. The light amount of the light source was corrected by using BS520 silicon photodiode of Bunkoh-Keiki Company.

As the result, electrochemical properties of the organic solar cell device are shown in FIG. 9, and summarized in Table 3.

[Example 6] Organic solar cell device using P3HT and Compound 2-6 as photoactive layer

The organic solar cell device was manufactured by the same method as Example 2, except that Compound 2-6 was used as an acceptor material of the photoactive layer, instead of Compound 2-1.

As the result, electrochemical properties of the organic solar cell device are shown in FIG. 9, and summarized in Table 3.

[Comparative example 2] Organic solar cell device using P3HT and PCBM as photoactive layer

The organic solar cell device was manufactured by the same method as Example 2, except that PCBM was used as an acceptor material of the photoactive layer, instead of Compound 1-1.

As the result, electrochemical properties of the organic solar cell device are shown in FIG. 9, and summarized in Table 3.

In general, power conversion efficiency of a solar cell may be calculated by the following Calculating equation 1.

[Calculating equation 1]

$$PCE(\%) = \frac{Voc \times Jsc \times FF}{Pinc}$$

[In Calculating equation 1, *Voc* is an open circuit voltage (V) and represents a voltage in the state while current does not flow; Jsc is short circuit current density (mA/cm$^2$) and represents current density at 0 V; FF is fill factor and represents a value of the maximum power value divided by a multiple of Voc and Jsc; *Pinc* represents intensity of light (mW/c$^2$) irradiated.

[Table 3] Comparison in characteristics of organic solar cell devices manufactured through mixing with P3HT

| Device | Compound | open circuit voltage Voc (mV) | short circuit voltage Jsc (mA/cm$^2$) | FF | PCE (%)* |
|---|---|---|---|---|---|
| Comparative example 1 | PCBM | 535 | 9.58 | 0.58 | 2.99 |
| Example 2 | Compound 2-1 | 828 | 7.61 | 0.64 | 4.02 |
| Example 3 | Compound 2-6 | 794 | 7.52 | 0.66 | 3.97 |
| * Measured under the conditions of AM 1.5 Isun (100mW/cm$^2$)<br>* After annealing at 150°**C** for 10 minutes | | | | | |

As shown in Table 3, it was confirmed that devices using the fullerene compound including an aromatic fused ring of the present invention have a higher Voc value as compared with the device using the existing PCBM. Particularly, in cases where di-adducts such as Compounds 2-1 and 2-6 were used as an electron acceptor material, high Voc values of 0.267 V and 0.276 V can be obtained respectively, which showed improved results by about 50%, as compared with an open circuit voltage of the organic solar cell device using PCBM as an electron acceptor material. Due to this improved open circuit voltage, a short-circuit current of each of the organic solar cell devices of the present invention was similar to that of the organic solar cell device using PCBM. However, it can be seen that power conversion efficiency of each of the organic solar cell device of the present invention was about 5%, while the organic solar cell device using PCBM was 2.99%.

In addition, as the measurement result of inner power conversion efficiency (IPCE), the device using Compound 2-1 as an electron acceptor material showed a relatively lower value at a region of 350 to 480 nm and a relatively higher value at a region of 570 to 650 nm, as compared with the device using PCBM as an electron acceptor material, and the maximum efficiency thereof was about 60%, which was similar therebetween. Through these results, it can be verified that short-circuit current (Jcs) between the two devices are similar (FIG. 10).

[Industrial Applicability]

Many materials for p-type organic thin film transistors are developed while materials for n-type organic thin film transistors are less known. The reason is that electron mobility is remarkably lower than hole transfer characteristic in an organic semiconductor material. Therefore, the fullerene derivatives of Chemical Formula 1 of the present invention can improve performance of an n-type organic thin film transistor, and can be easily used in manufacturing devices through a solution process due to excellent solubility thereof, and thus, it will be expected to be commercially useful. The fullerene derivatives of Chemical Formula 2 of the present invention can be easily synthesized. Further, they are

n-type organic semiconductor materials having excellent electron mobility, and they are used as an acceptor material of the organic solar cell device to provide a device having high an open circuit voltage (Voc), thereby improving power conversion efficiency of the organic solar cell device. Further, they are materials suitable for a low-cost printing process due to excellent solubility thereof, and thus they are expected to be appropriate in manufacture of a large-area high-efficiency organic solar cell device.

**Claims**

1. A fullerene derivative represented by Chemical Formula 1 below:

[Chemical Formula 1]

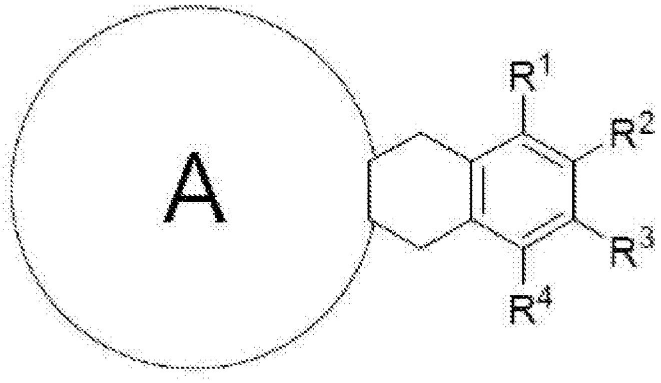

[In Chemical Formula 1, $R^1$ through $R^4$ are independently selected from a hydrogen atom and linear or branched chain (C1-C20)alkyl or linked to an adjacent substituent via (C4-C8)alkenylene to form an aromatic fused ring, or the alkenylene is substituted with one to three hetero atoms selected from an oxygen atom, a nitrogen atom, and a sulfur atom to form a hetero aromatic fused ring; and A represents fullerene of C60 or C70.]

2. The fullerene derivative of claim 1, wherein in Chemical Formula 1, $R^1$ through $R^4$ are independently selected from hydrogen and methyl, or $R^2$ and $R^3$ are linked via C4 alkenylene to form an aromatic fused ring.

3. The fullerene derivative of claim 1, wherein the compound of Chemical Formula 1 is selected from the following compounds.

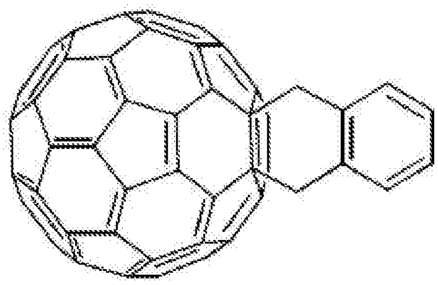

1-1

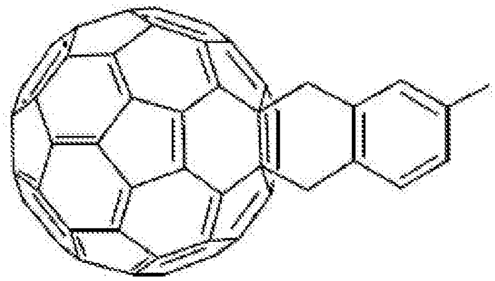

1-2

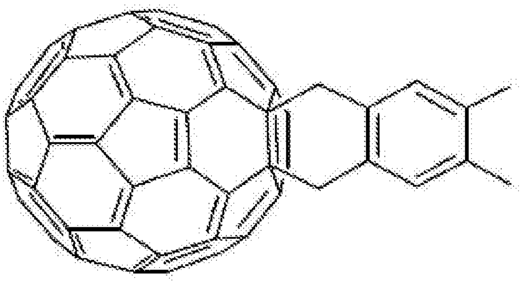

1-3

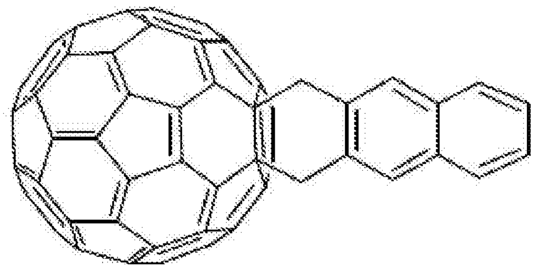

1-4

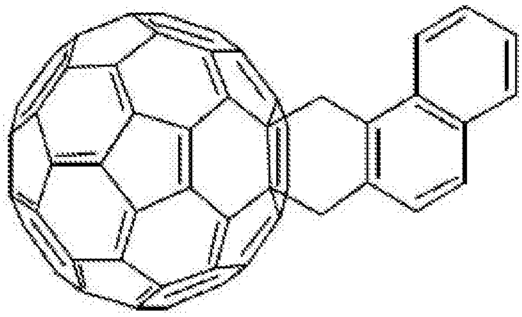

1-5

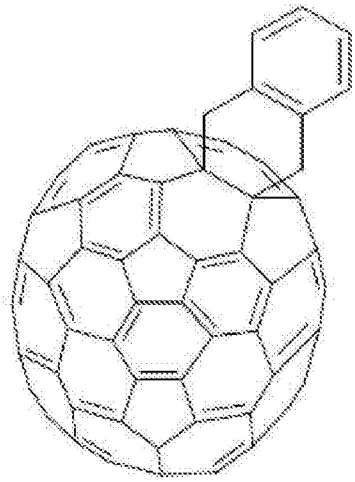

1-6

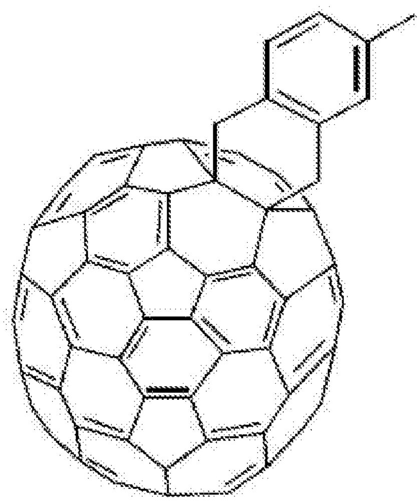

1-7

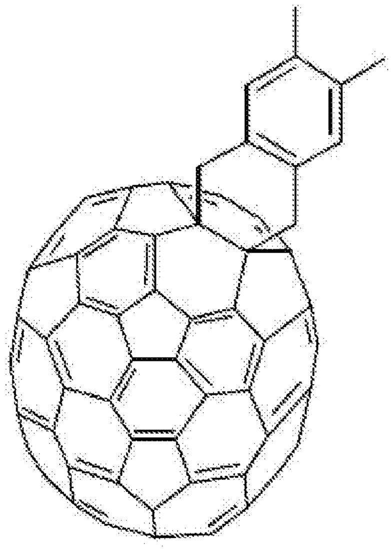

1-8

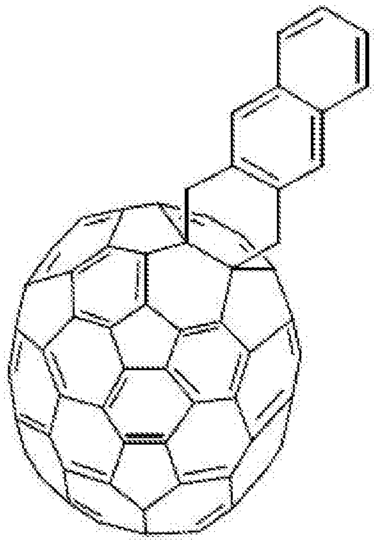

1-9

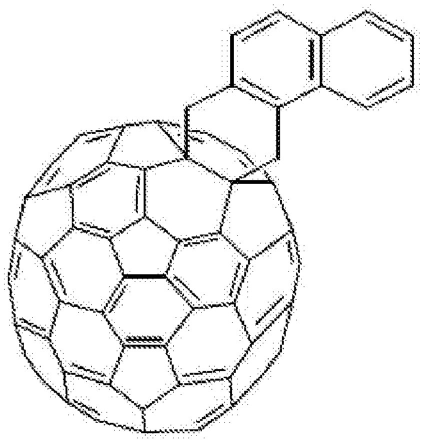

1-10

**4.** A fullerene derivative represented by Chemical Formula 2 below:

[Chemical Formula 2]

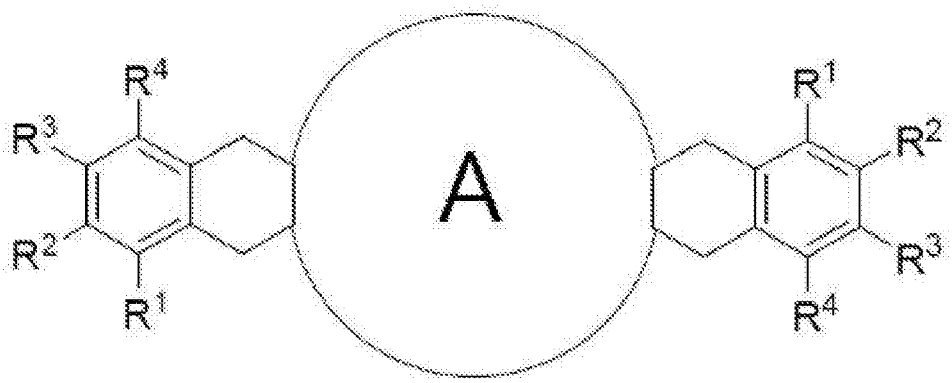

[In Chemical Formula 2, $R^1$ through $R^4$ independently are selected from a hydrogen atom and linear or branched chain (C1-C20)alkyl or linked to an adjacent substituent via (C4-C8)alkenylene to form an aromatic fused ring, or the alkenylene is substituted with one to three hetero atoms selected from an oxygen atom, a nitrogen atom, and a sulfur atom to form a hetero aromatic fused ring; and A represents fullerene of C60 or C70.]

**5.** The fullerene derivative of claim 4, wherein in Chemical Formula 2, $R^1$ through $R^4$ are independently selected from hydrogen and methyl, or $R^2$ and $R^3$ are linked via C4 alkenylene to form an aromatic fused ring.

**6.** The fullerene derivative of claim 4, wherein the compound of Chemical Formula 2 is selected from the following compounds.

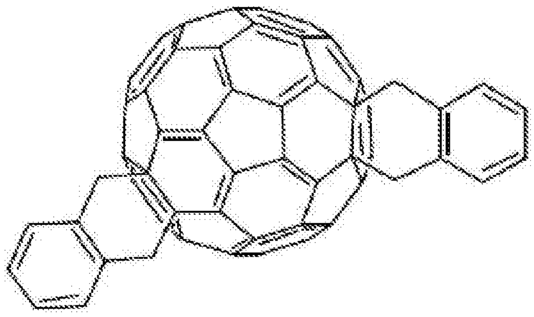

2-1

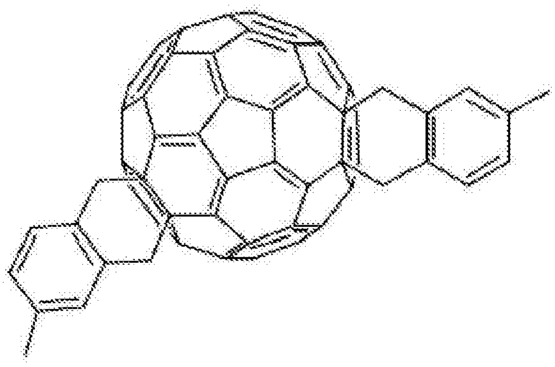

2-2

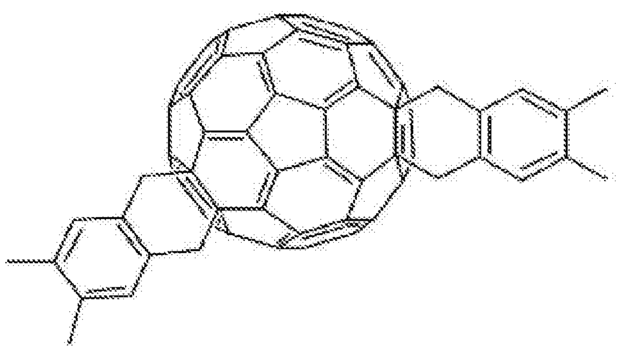

2-3

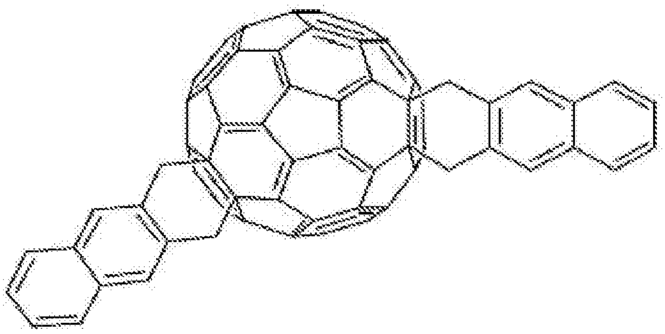

2-4

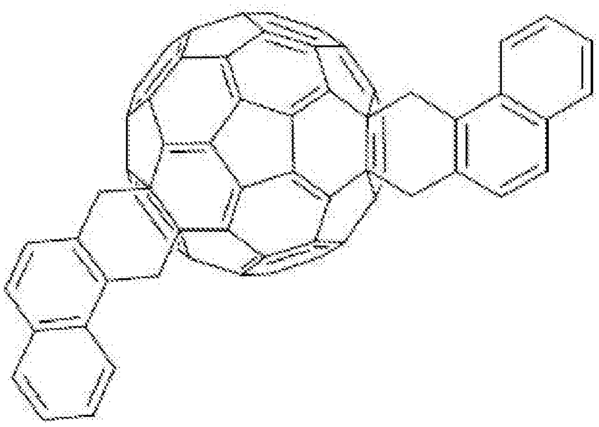

2-5

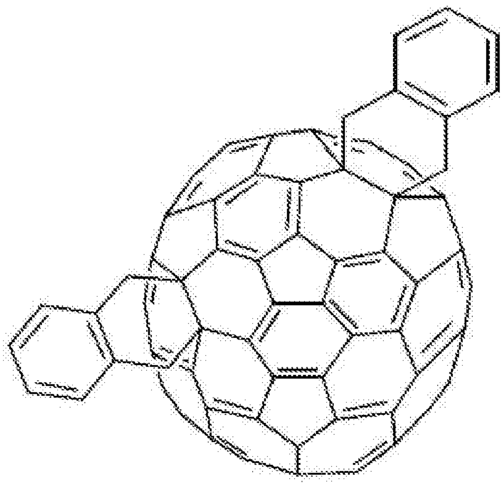

2-6

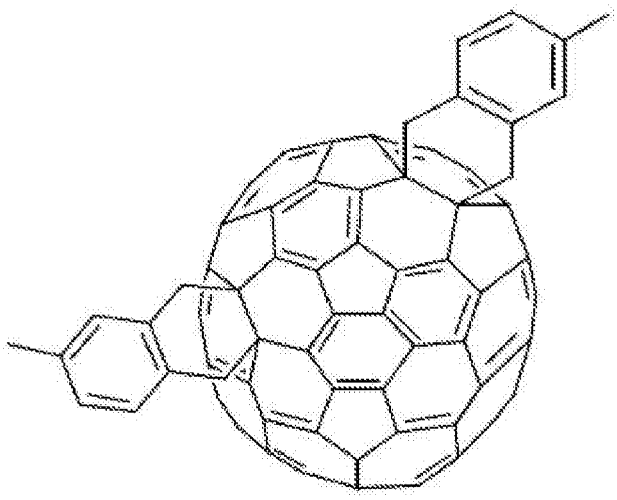

2-7

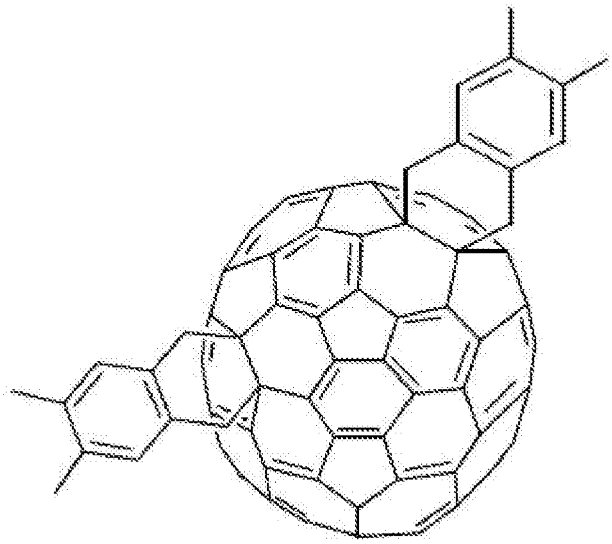

2-8

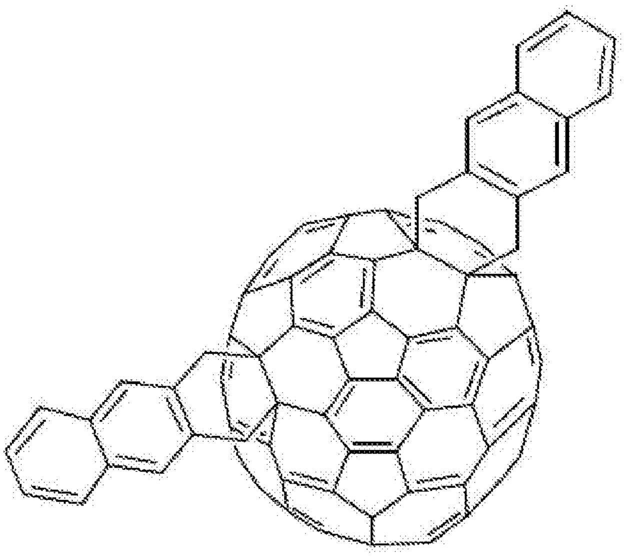

2-9

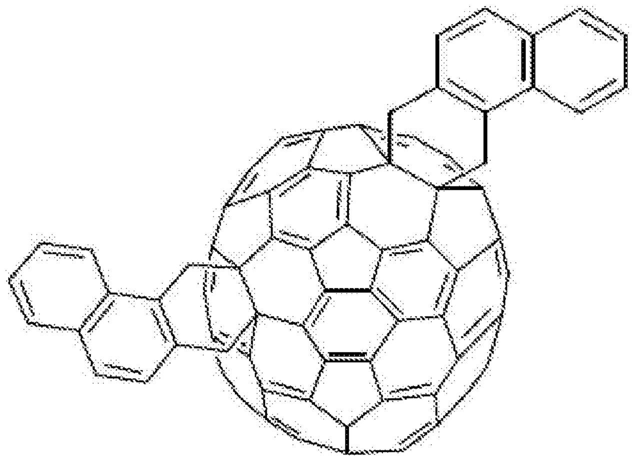

2-10

7. An organic thin film transistor comprising the fullerene derivative of any one of claims 1 to 3.

8. The organic thin film transistor of claim 7, wherein the fullerene derivative is used as a channel material.

9. The organic thin film transistor of claim 7, wherein the fullerene derivative is formed by a solution process method or a deposition method.

10. An organic solar cell device comprising the fullerene derivative of any one of claims 1 to 3.

11. The organic solar cell device of claim 10, wherein the fullerene derivative is used as an acceptor material.

12. The organic solar cell device of claim 10, wherein the fullerene derivative is formed by a solution process method or a deposition method.

【FIG. 1】

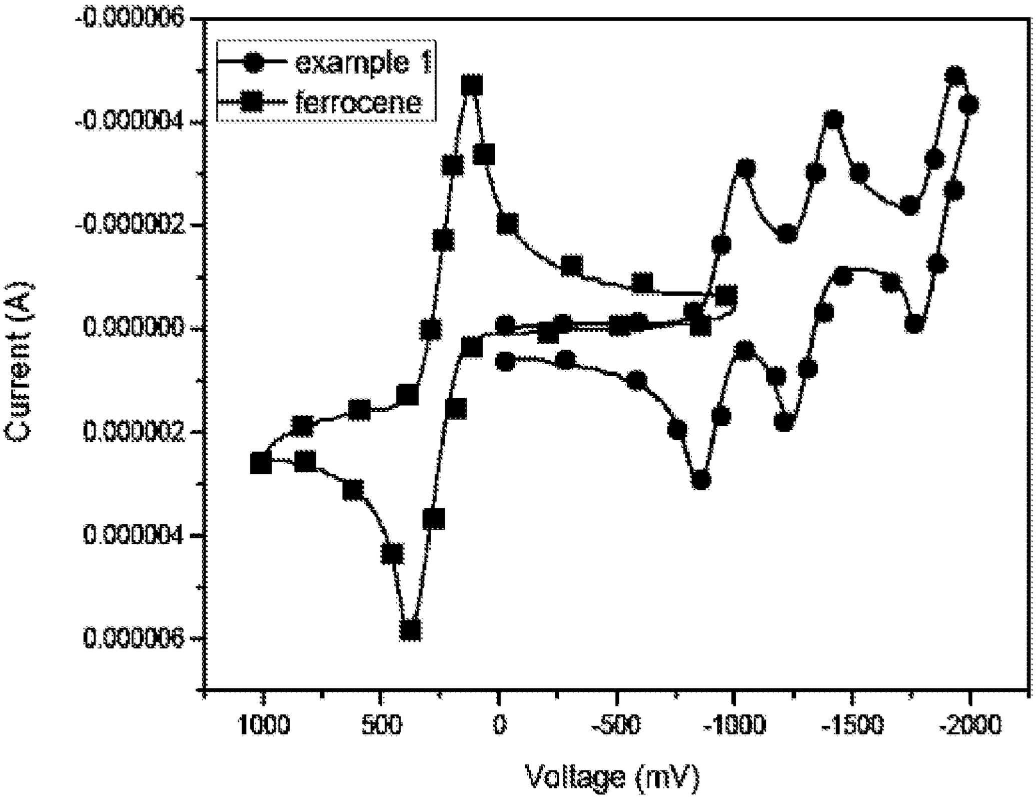
example 1
ferrocene
-0.000006
-0.000004
-0.000002
0.000000
0.000002
0.000004
0.000006
Current (A)
1000
500
0
-500
-1000
-1500
-2000
Voltage (mV)

【FIG. 2】

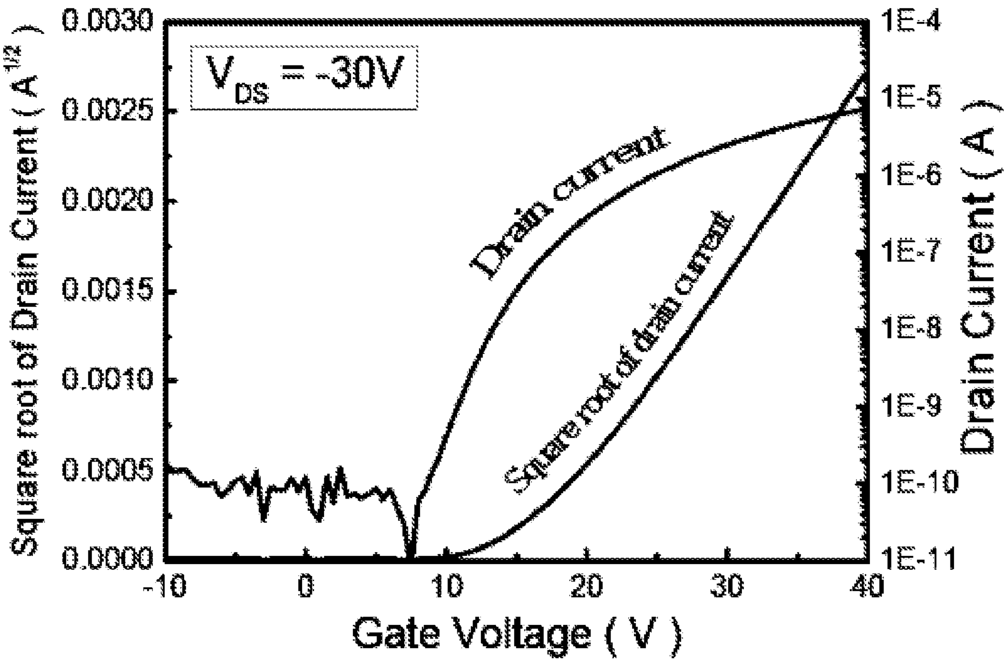

V_DS = -30V
Drain current
Square root of drain current
Square root of Drain Current ( A^1/2 )
Drain Current ( A )
Gate Voltage ( V )
0.0030
0.0025
0.0020
0.0015
0.0010
0.0005
0.0000
1E-4
1E-5
1E-6
1E-7
1E-8
1E-9
1E-10
1E-11
-10
0
10
20
30
40


【FIG. 3】

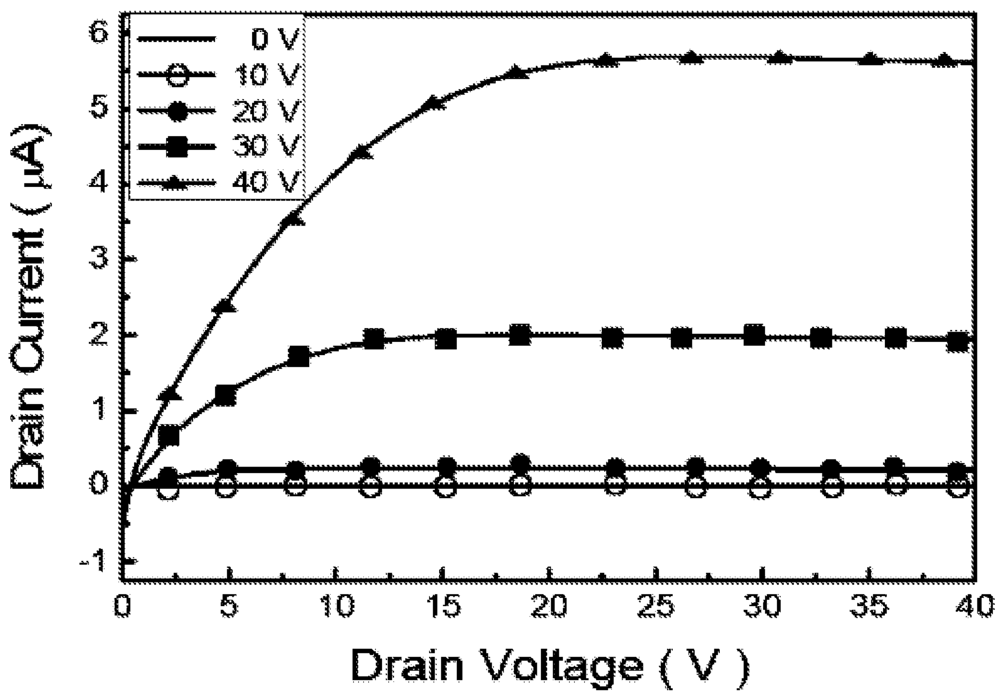

0 V
10 V
20 V
30 V
40 V
Drain Current ( μA)
Drain Voltage ( V )
6
5
4
3
2
1
0
-1
0
5
10
15
20
25
30
35
40

【FIG. 4】

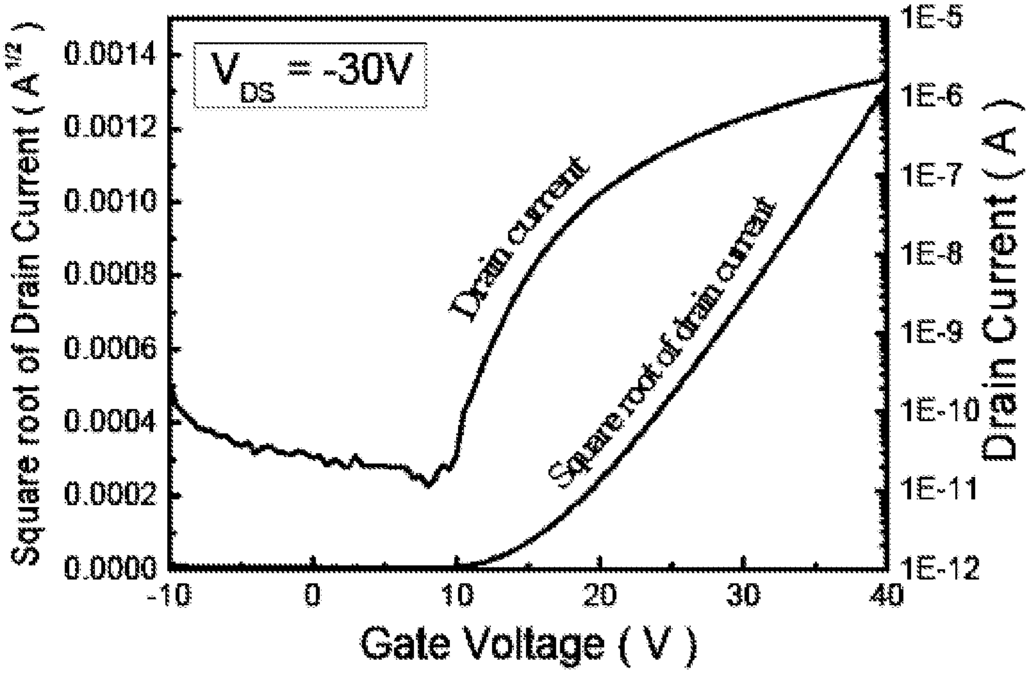

$V_{DS}$ = -30V
Drain current
Square root of drain current
Square root of Drain Current ( A 1/2 )
0.0000
0.0002
0.0004
0.0006
0.0008
0.0010
0.0012
0.0014
Drain Current ( A )
1E-12
1E-11
1E-10
1E-9
1E-8
1E-7
1E-6
1E-5
-10
0
10
20
30
40
Gate Voltage ( V )


【FIG. 5】

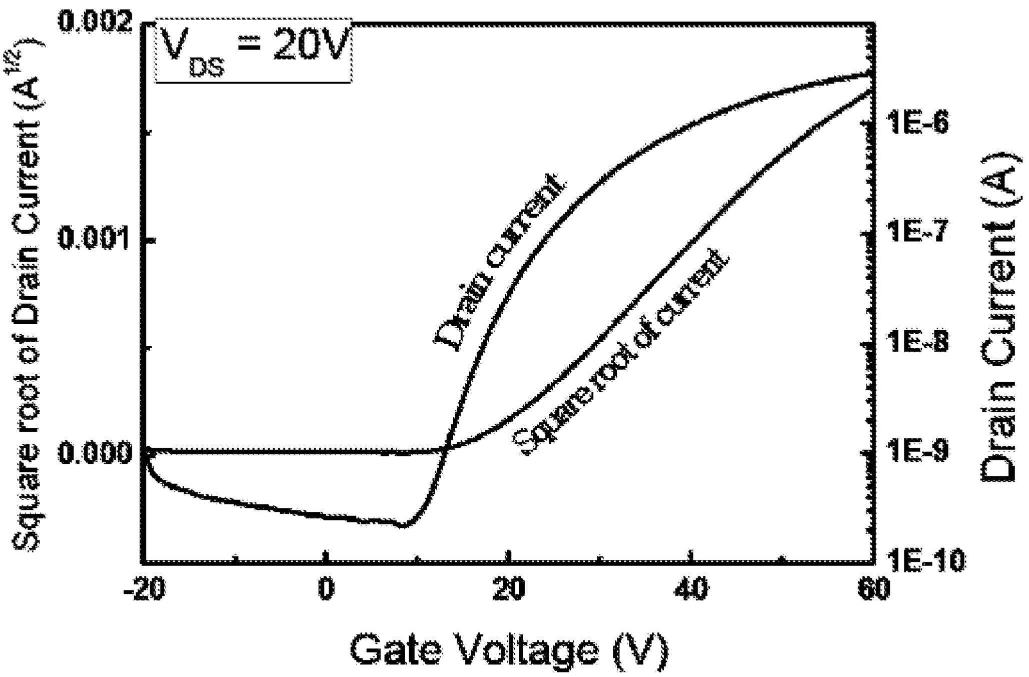

$V_{DS}$ = 20V
Drain current
Square root of current
Square root of Drain Current (A 1/2)
0.000
0.001
0.002
Drain Current (A)
1E-10
1E-9
1E-8
1E-7
1E-6
-20
0
20
40
60
Gate Voltage (V)

【FIG. 6】

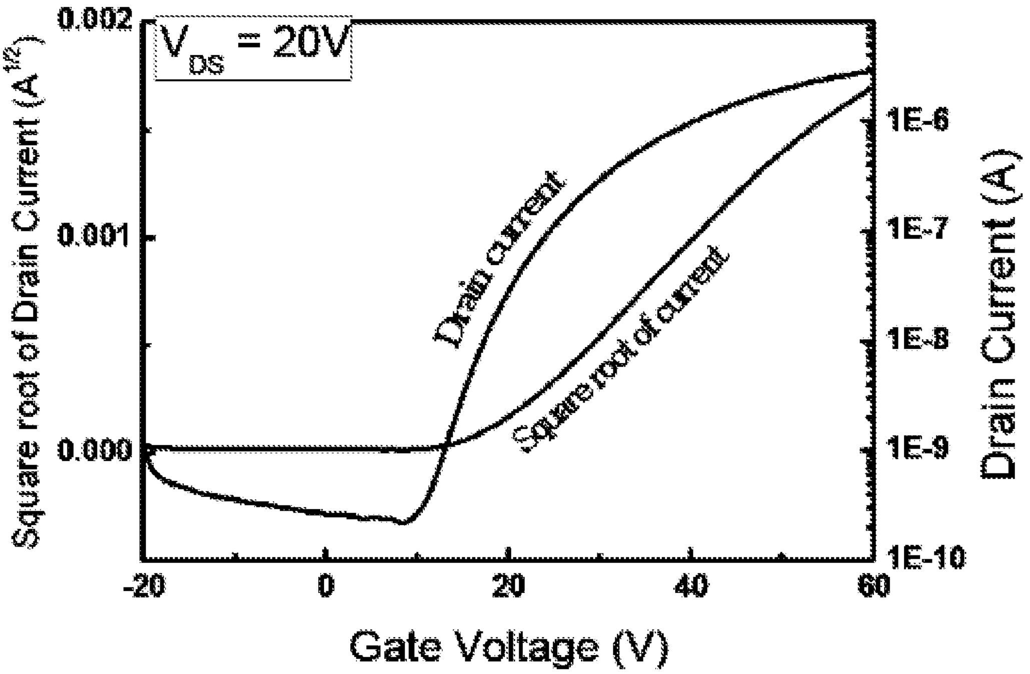

V_DS = 20V
Drain current
Square root of current
Square root of Drain Current (A^1/2)
0.002
0.001
0.000
1E-6
1E-7
1E-8
1E-9
1E-10
Drain Current (A)
-20
0
20
40
60
Gate Voltage (V)


【FIG. 7】

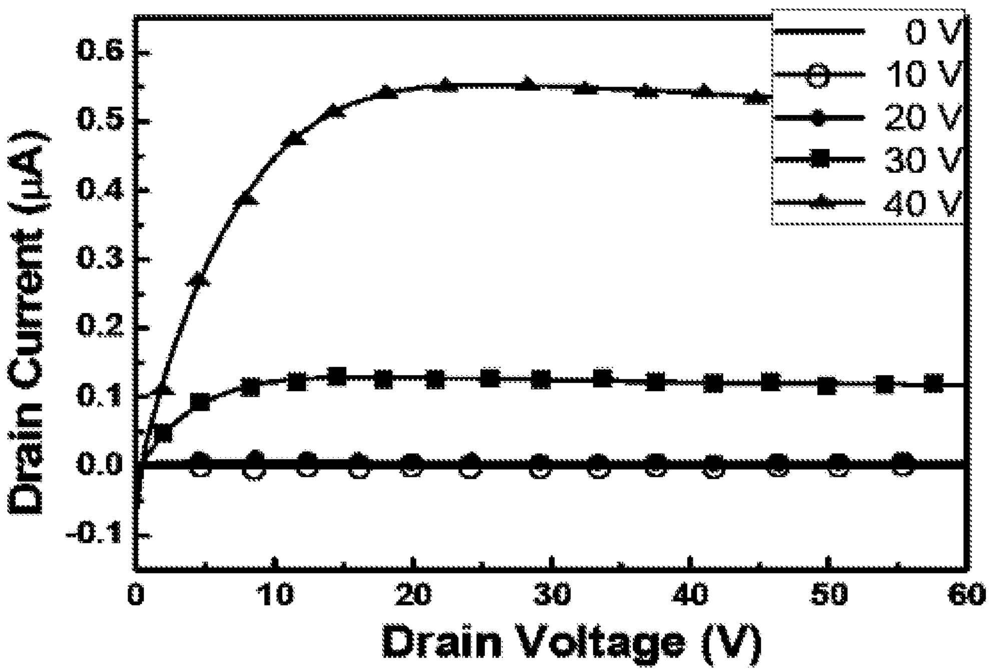

0 V
10 V
20 V
30 V
40 V
0.6
0.5
0.4
0.3
0.2
0.1
0.0
-0.1
Drain Current (μA)
0
10
20
30
40
50
60
Drain Voltage (V)

【FIG. 8】

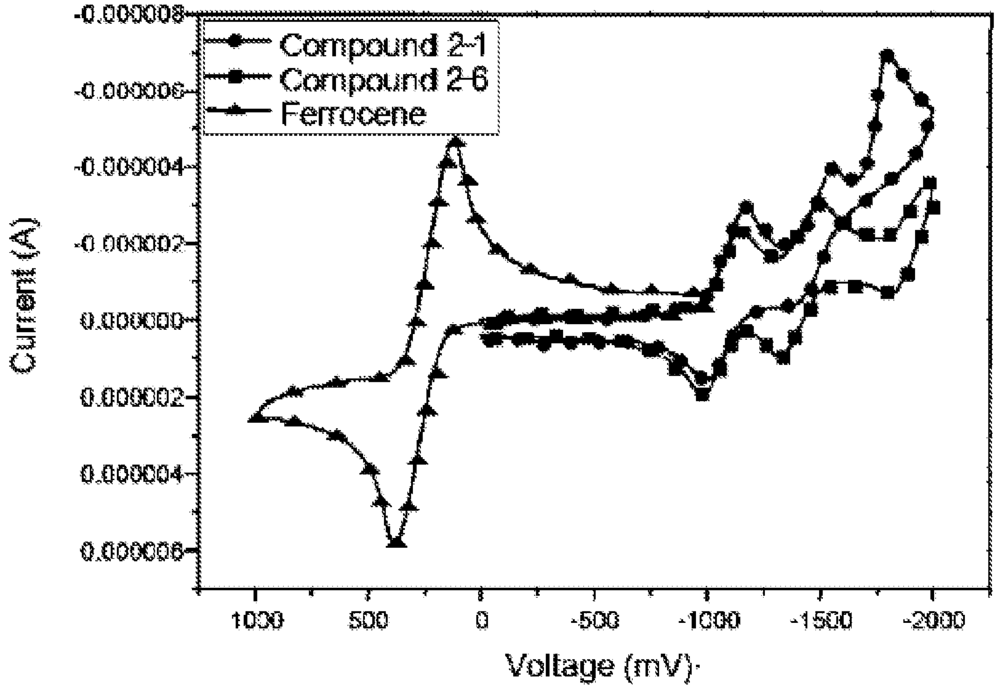
Compound 2-1
Compound 2-6
Ferrocene
Current (A)
Voltage (mV)
-0.000008
-0.000006
-0.000004
-0.000002
0.000000
0.000002
0.000004
0.000006
1000
500
0
-500
-1000
-1500
-2000

【FIG. 9】

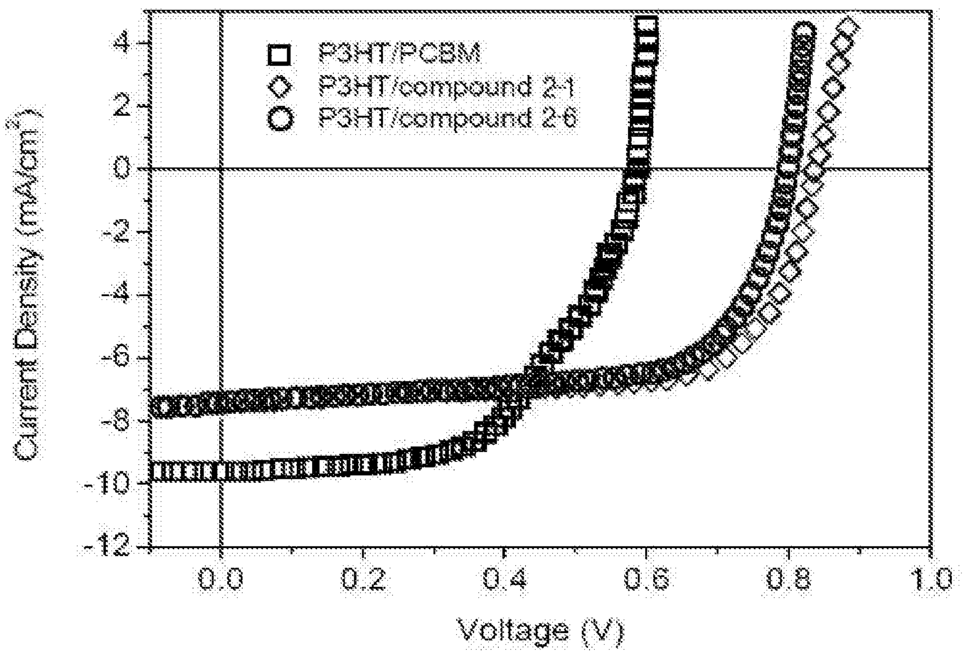
P3HT/PCBM
P3HT/compound 2-1
P3HT/compound 2-6
Current Density (mA/cm²)
Voltage (V)
4
2
0
-2
-4
-6
-8
-10
-12
0.0
0.2
0.4
0.6
0.8
1.0

【FIG. 10】

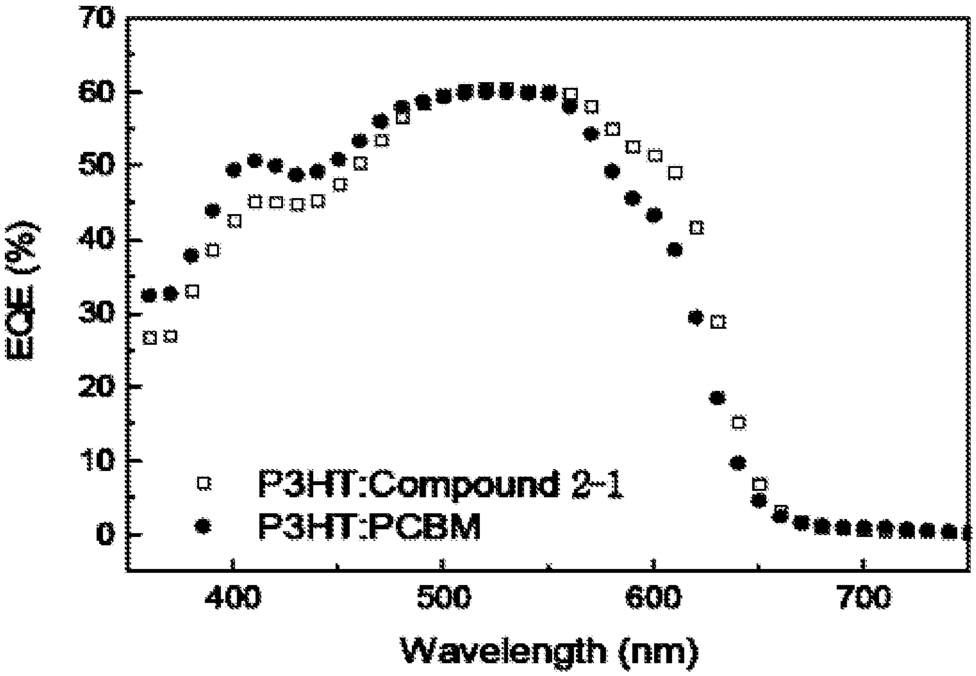
EQE (%)
Wavelength (nm)
P3HT:Compound 2-1
P3HT:PCBM
0
10
20
30
40
50
60
70
400
500
600
700

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **C.J. Brabec et al.** *Adv. Func. Mater.,* 2001, vol. 11, 374